# EUROPEAN PATENT SPECIFICATION

(11) **EP 1 759 597 B1**
(45) Date of publication and mention of the grant of the patent: **21.01.2009**
(21) Application number: 06025815.9
(22) Date of filing: 27.02.2004
(51) Int. Cl.: A23L 1/30, A61K 35/74, A23C 9/123

(54) **Probiotic composition**
Probiotische Zusammensetzung
Composition probiotique

(30) Priority: 13.03.2003 JP 2003068943; 14.10.2003 JP 2003354277
(43) Date of publication of application: 07.03.2007
(62) Divisional of application: 04715523.9
(73) Proprietor: Kirin Holdings Kabushiki Kaisha, Tokyo (JP)
(72) Inventor: Inoue, Sayo, c/o Kirin Beer K.K., Yokohama-shi, Kanagawa 236-0004 (JP); Fujii, Toshio, c/o Kirin Beer K.K., Yokohama-shi, Kanagawa 236-0004 (JP); Fujiwara, Daisuke, c/o Kirin Beer K.K., Yokohama-shi, Kanagawa 236-0004 (JP); Saiki, Akira, c/o Kirin Beverage Corporation Res.& Dev. Lab.,, Koza-gun, Kanagawa 253-0101 (JP); Takahashi, Minoru, c/o Kirin Beverage Corporation Res.& Dev. Lab.,, Koza-gun, Kanagawa 253-0101 (JP); Yamauchi, Koichiro, c/o Kirin Beverage Corporation Res.& Dev. Lab.,, Koza-gun, Kanagawa 253-0101 (JP); Gotou, Masami, c/o Koiwai Dairy Products Co., Ltd., Tokyo 100-0005 (JP); Nishida, Satoshi, c/o Koiwai Dairy Products Co., Ltd., Tokyo 100-0005 (JP); Deuchi, Keiji, c/o Kirin Well Foods Company, Ltd., Tokyo 135-0044 (JP); Wakabayashi, Hideyuki, c/o Kirin Beer K.K., Yokohama-shi, Kanagawa 236-0004 (JP); Komeda, Toshihiro, c/o Kirin Beer K.K., Yokohama-shi, Kanagawa 236-0004 (JP)
(74) Representative: HOFFMANN EITLE

(56) References cited:
- EP-A- 1 034 788
- EP-A- 1 364 586
- WO-A-02/18542
- WO-A-02/28402
- INOUE SAYO ET AL: "Ko-allergy koka no aru nyusankin Lactobacillus paracasei KW3110-kabu no senbatsu" JAPAN SOCIETY FOR BIOSCIENCE, BIOTECHNOLOGY AND AGROCHEMISTRY TAIKAI KOEN YOSHISHU, 5 March 2003 (2003-03-05), page 74, XP002984613
- INOUE SAYO ET AL: "Nyusankin Lactobacillus paracasei KW3110-kabu no dobutsu model deno koka" JAPAN SOCIETY FOR BIOSCIENCE, BIOTECHNOLOGY AND AGROCHEMISTRY TAIKAI KOEN YOSHISHU, 5 March 2003 (2003-03-05), page 75, XP002984614
- "Kalpis, kirin, kafunsho ya allergy ni taisuru nyusankin koka o aitsugi nogei kagakukai de happyo" NIKKEI BIOTECHNOLOGY, 3 March 2003 (2003-03-03), XP002984615
- BONETTI ANTONELLA ET AL: "ASSESSMENT OF THE PERSISTENCE IN THE HUMAN INTESTINAL TRACT OF TWO PROBIOTIC LACTOBACILLI LACTOBACILLUS SALIVARIUS I 1794 AND LACTOBACILLUS PARACASEI I 1688." MICROBIAL ECOLOGY IN HEALTH & DISEASE, CHICHESTER, GB, vol. 14, no. 4, November 2002 (2002-11), pages 228-232, XP009075612
- DATABASE WPI Week 200281 Derwent Publications Ltd., London, GB; AN 2002-747895 XP002420230 & KR 2002 023 886 A (PARK S) 29 March 2002 (2002-03-29)
- KRISTO E ET AL: "Modelling of rheological, microbiological and acidification properties of a fermented milk product containing a probiotic strain of Lactobacillus paracasei." INTERNATIONAL DAIRY JOURNAL 13 (7) 517-528 2003 CORRESPONDENCE (REPRINT) ADDRESS, C. G. BILIADERIS, DEP. OF FOOD SCI. & TECH., FAC. OF AGRIC., ARISTOTLE UNIV., GR-540 06 THESSALONIKI, GREECE. TEL. +30 2310 998785. FAX +30 2310 471257. E-MAIL BI, 2003, XP002418020

## Description

### Technical Field

The present invention relates to a probiotics product, a method for said product, and moreover to use of lactic acid bacteria for producing said product.

### Background Art

Allergy is one of the diseases most frequently occurred in the developed countries. The development mechanism of allergy is generally_classified into four classes, that is type I to type IV. Type I allergy associated with IgE antibodies are typified by hay fever, asthma, urticaria, anaphylaxy shock and the like. Type II allergy associated with IgG antibodies and IgM antibodies is a cytotoxic reaction which activates complement systems, and fetal erythroblastosis and autoimmune hemolytic anemia belong to this type. Type III allergy is a tissue injury caused by antigen-antibody complex, and is a reaction typified by Alex reaction, serum sickness, glomerulonephritis and the like. Type IV allergy is a delayed allergy (delayed hypersensitivity) associated with T cells, typified by tuberculin reaction or contact dermatitis. It is said that type I, II and IV among allergy are associated with the development of food allergy. On the other hand, it is known that environmental allergy, that is hay fever, atopic dermatitis, bronchial asthma, chronic allergic rhinitis, allergic conj unctivitis and the like have mainly the development mechanism of type I.

Type I allergy mentioned above is characterized by induction of allergen-specific IgE, release of chemical mediators such as histamine, leukotriene and the like. Immune response occurs from interaction of various cells, and helper T cells (Th) are one of the types of cells involved in this reaction. Helper T cells being classified into subgroups of T cells, generate various cytokines (helper factors) by recognizing antigens, and control the induction of immune response. Helper T cells are classified into Th1 cells and Th 2 cells based on their cytokine producing ability. Th2 cytokines such as IL-4, IL-5, IL-13 and the like are necessary, so that class switch of antibody occurs in B cells to generate IgE antibodies. Actually, it is known that Th2 cells are increased in lymphocytes of allergic patients. In other words, allergens that have penetrated from the outer world, are presented to T cells, in a conditions that a part is bound to MHC class II molecules by antigen presenting cells such as dendritic cells, macrophages and the like, and then Th2 cells are activated and differentiated. Th2 cytokines released by Th2 cells induce class switch of B cells, IgE antibodies are generated, and the IgE antibodies are bound with mast cells in the tissues and FcR on the surface of basophils in blood. Allergens are recognized by IgE antibodies bound to mast cells or on the surface of basophils at the time of the next invasion, and crosslinking are formed between IgE antibodies. This stimulation as a trigger, mast cells and basophils release significantly the chemical mediators, and various symptoms of allergy appear.

As for prevention and treatment of allergy through IgE or chemical mediators, examples include: antihistamine that inhibit signaling from the peripheral nerve by binding in an antagonistic manner with histamine to histamine receptor; antiallergic drug for trying to relief symptoms by diminishing the activity of chemical mediators-producing cells; steroid relieving inflammation by diminishing immune response; hyposensitization therapy inducing tolerance by injecting periodically the allergen itself. However, side effects are being problems for all of these, and none of these have definitive effects.

Recently, a method for controlling the production of Th2 cytokine is getting attention for the purpose of suppressing IgE. Certain bacterium such as *tubercle bacilli,* hemolytic *streptococcus,* the lactic acid bacteria and the like have been reported to enhance Th1 immunity and suppress Th2 immunity to lower IgE as a result (Clinical Immunology, vol. 32, p. 454; International Archives of Allergy and Immunology, vol. 115, p. 278, 1998; Japanese Laid-Open Patent Application No.9-2959).

Among these, the lactic acid bacteria being easy to apply to foods from the point of safety, are usefulmaterials . However, as for the lactic acid bacteria, it is not appropriate to say that all lactic acid bacteria have strong effect to enhance Th1 immunity, and it is indispensable to select useful bacterial strains. As for antiallergic lactic acid bacteria, *L. rhamnosus* LGG strain is publicly known and it is reported that by administering to expectant mothers, the development of atopic dermatitis of the child is suppressed (Lancet, vol. 357, p. 1076, 2001). The use of the lactic acid bacteria as antiallergical drugs is disclosed in several Patent Gazettes. For example, Japanese Laid-Open Patent Application No. 9-2959 discloses the use of the lactic acid bacteria as antiallergic drug wherein IgE production level is 30 ng/ml or less when the lactic acid bacteria such as *L. acidophilus, L. brevis, L. buchnerii, L. casei* and the like are added to culture mouse lymphocytes; Japanese Laid-Open Patent Application No. 10-309178 discloses the use of bifidobacteria such as *Bifidobacterium. infantis, Bifidobacterium. breve, Bifidobacterium. longum, Bifidobacterium. bifidum* and the like as antiallergic drug to treat especially food allergy; and Japanese Laid-Open Patent Application No. 2000-95697, the use of lacid acid bacteria such as *Enterococcus faecalis, Lactobacillus leuteri* and the like, as inhibitor of type I allergy such as allergic bronchial asthma, chronic allergic rhinitis, atopic dermatitis and the like.

On the other hand, it is known that the enhancement of Th1 immunity by the lactic acid bacteria and the like relates to the activation of cellular immunity such as macrophage, killer T cells (CD8⁺ T cells), NK cells through the production of IL12, IFN.γ and it is known that it leads to the resistance to viral or bacterial infection, or development of cancer. Specifically, IL-12 produced by macrophage, leads to the resistance against foreign enemy, cancer through differentiation of the naive helper T (Th0) cells to Th1 cells, activation of monocytes, macrophage or NK cells. Therefore, it is suggested that the lactic acid bacteria strain being able to induce strong IL-12 production can be used as immunoadjuvant (Cancer Immunology Immunotherapy, vol. 49, p. 157, 2000; Japanese Laid-Open Patent Application No. 7-228536, Japanese Laid-Open Patent Application No. 2002-80364).

Furthermore, it is said that the lactic acid bacteria should be resistant to digestive juice such as gastric acid, bile acid so that the lactic acid bacteria can reach the intestinal tract alive and to be effective in various ways (Symposium on Intestinal Flora 3, "Intestinal Flora and probiotics", Gakkai Shuppan Center, p.41-55, edited by Tomotari Mitsuoka 1998). Moreover, it is known that the lactic acid bacteria highly adhesive to intestinal tract are fully effective by remaining in the intestinal tract. Therefore, in case of ingesting the lactic acid bacteria orally, it is preferable that the lactic acid bacteria reach the intestinal tract alive, besides being highly functionality.

Recently, there are many people whose constitution of their body has changed, such as atopic dermatitis or hay fever, and it seems to be caused by allergy. This is becoming a social problem. Among these persons, treatment using various drugs is provided to patients having particularly severe symptoms. However, most of the patients have not reached the point to receive full-scale treatment, and as actual situation, they are receiving symptomatic therapy to keep daily life, including folk remedy.

In view of this situation, recently, many researches are conducted actively targeting to suppress allergy by diet. As a result, antiallergic effects are found in ingredients of various foods or drinks such as Japanese basil oil, fish oil, particular tea polyphenol and the like, and treatment by using these are provided.

As ingredients of foods and beverages having antiallergic effect, similar effects in particular the lactic acid bacteria are now known such as described above, and some yogurts or the lactic acid bacteria beverages using the lactic acid bacteria are practically used as foods or drinks having antiallergic effect. As for prevention or treatment using this kind of antiallergic foods and drinks, it is possible to ingest under safe and mild conditions but it is necessary to ingest these every day continuously, and it is also necessary to be effective with the amount possible to ingest continuously. However, conventionally, since a method for obtaining and selecting microbial strains with high antiallergic activity has not been clarified, any promising microbial strain was not obtained, thus it was not a satisfactory method for practical use. Moreover, in conventional use of the lactic acid bacteria, the main target was fermentation by the lactic acid bacteria as in yogurts or the lactic acid bacteria beverages, and antiallergic effects were treated as secondary effects. Furthermore, in case of yogurts and the lactic acid bacteria beverages, as it is indispensable to transport under chilling temperature, and many of these are not keeping quality for a long term, there are problems that there is a limit of handling, it was not satisfactory as foods or drinks to be used for real prevention or treatment of allergy that is possible to ingest every day continuously, and of great efficacy. Therefore, the development of antiallergic composition of great efficacy that can solve these problems, and foods or drinks or the like using these are highly requested recently.

Inoue Sayo et al., Japan Society for Bioscience, Biotechnology and Agrochemistry Taikai Koen Yoshishu, 5 March 2003, page 74 relates to Lactobacillus paracasei KW3110 having antiallergic activity. The same is true for Inoue Sayo et al., Japan Society for Bioscience, Biotechnology and Agrochemistry Taikai Koen Yoshishu, 5 March 2003, page 75, and Nikkei Biotechnology, 3 March 2003, XP002984615.

The object of the present invention is to provide a probiotics product, a method of producing a probiotics product and the use of a viable lactic acid bacteria Lactobacillus paracasei KW3110 strain or its mutant for such a production. "With antiallergic function" above mentioned relates to "be effective to prevention/treatment of allergy and/or to improve/relief allergic symptoms".

The present inventors searched the active ingredients by targeting the prevention/treatment of environmental allergy such as hay fever/atopic dermatitis, bronchial asthma, chronic allergic rhinitis and the like, noted the lactic acid bacteria from the point of safety, made a keen study, selected the lactic acid bacteria that inhibits Th2 activation and activates Th1 on an appropriate balance, and found out that it is possible to obtain the lactic acid bacteria with high allergic activity.

In other words, a reference method for obtaining the lactic acid bacteria with high allergic activity is characterized by being a method focusing on Th1/Th2 balance. Conventional methods estimate antiallergic activity by measuring IgE. It is no doubt that IgE is associated with allergy, but on the other hand, for example, as it is understood that there are atopic patients with high IgE or low IgE, it is also a fact that everything cannot be explained with IgE level. It is known that various phenomena beside IgE are associated comprehensively with the development of allergy, such as increase of eosinophils or release of chemokines inducing invasion of lymphocytes to the spot. Therefore, the balance between Th1/Th2 that explains comprehensively about increase of IgE, increase of eosinophils or about chemokines are focused, and by using the balance as an index, a method for estimating and separating antiallergic microbial strains was developed. Thus, the lactic acid bacteria with high antiallergic activity were successfully obtained.

Such a reference antiallergic composition comprises antiallergic composition having the lactic acid bacteria with a particular balance between production level of interleukin 4 (IL-4) being an index of Th2 immunity activation and interleukin 12(IL-12) being an index of Th1 immunity activation as active ingredients, showing equal interleukin 12 production level compared to when *L. paracasei* KW3110 strain is used as a lactic acid bacterium to be tested, or showing 60% or more of interleukin 12 production level compared to when *L. paracasei* KW3110 strain is used, and showing less than 50% of interleukin 4 production level of a control wherein the lactic acid bacteria are not added, in case lymphocytes derived from mouse spleen sensitized with ovalbumin are suspended in a medium containing ovalbumin, and cultured by adding the lactic acid bacteria to be tested. Thus, the lactic acid bacteria with antiallergic activity can encompass: the lactic acid bacteria showing 70% or more of interleukin 12 production level compared to the KW3110 strain; the lactic acid bacteria showing 70% or more of interleukin 12 production level, and less than 40% of interleukin 4 production level as active ingredients, moreover the lactic acid bacteria showing equal or higher antiallergic activity compared to KW3925; the lactic acid bacteria showing equal or higher antiallergic activity compared to KW4110; the lactic acid bacteria showing equal or higher antiallergic activity compared to KW3926, when compared to the lactic acid bacteria KW4110, KW3925 and KW3926 with antiallergic activity that are obtained as active ingredients.

*L. paracasei* KW3110 strain that is the active ingredient of the probiotics antiallergic composition of the present invention, is resistant to digestive juice, is a lactic acid bacterium highly adhesive to intestinal tract, and shows particularly an excellent effect when administered orally as antiallergic drug.

*L. paracasei* KW3110 used as active ingredients of the probiotics antiallergic composition in the present invention is deposited as FERM BP-08634 in National Institute of Advanced Industrial Science and Technology, International Patent Organism Depositary, which is an International Depositary Authority, according to Budapest Treaty on the deposit of microorganism for patent procedure.

As for the use of the probiotics product of the present invention as foods or drinks, it encompasses particularly beverages such as tea drinks, and beverages with antiallergic function. Beverages with antiallergic function of the present invention can have good taste and have a long shelf life, and it is possible to prepare as a beverage possible to ingest everyday continuously, and having antiallergic function that can become effective with the amount possible to ingest continuously. In the present invention, the above mentioned lactic acid bacteria can be used being sterilized by a treatment such as heat sterilization. For example, it is possible to merchandize as a beverage filled in a sealed container that can maintain the flavor of the beverage itself, that is stabilized as a beverage product, and that can maintain the antiallergic function, by adding the lactic acid bacteria of the present invention after being sterilized by heating, and manufactured as a beverage in a sterilized, sealed container. Moreover, when manufacturing as a beverage in a sealed container, by adjusting the concentration of the lactic acid bacteria to be used, it is possible to manufacture a beverage having good taste and long shelf life by enhancing the function of maintaining the flavor of the beverage itself as well as maintaining the antiallergic function. In the present invention, it is preferable from the point of view of antiallergic activity, storage of the product and stabilization, to produce various types of nondairy beverages, that is, nondairy soft drinks containing juice or tea drinks.

As for the use of the probiotics product of the present invention to foods or drinks, two or more types of lactic acid bacteria with high antiallergic activity can be used by being combined. At that time, the combination or the content of compositions can be adjusted according to each food or drink. Further, it can also compound or combined with other antiallergic compositions.

### Disclosure of the Invention

In other words, the present invention relates to :
1. A probiotics product comprising a viable lactic acid bacteria Lactobacillus paracasei KW3110 strain or its mutant as an active ingredient, wherein the probiotics product is a beverage or food product;
2. The probiotics product according to item 1, wherein the probiotics product is a dairy raw material fermented by using the lactic acid bacteria Lactobacillus paracasei KW3110 strain or its mutant;
3. The probiotics product according to item 1 to 2, wherein the probiotics product is yogurt;
4. The probiotics product according to item 1 to 3, wherein the number of the lactic acid bacteria Lactobacillus paracasei KW3110 strain or its mutant in the probiotics- product is 5 X 10⁹ or more cells per daily intake;
5. The probiotics product according to item 1 to 4, wherein the probiotics product is used for preventing and/or treating allergic diseases;
6. A method for producing probiotics products, wherein a part or whole of the lactic acid bacteria in the beverage or food produced by using lactic acid bacteria or the beverage or food comprising lactic acid bacteria is replaced by the lactic acid bacteria Lactobacillus paracasei KW3110 strain or its mutant;
7. A method for producing a probiotics product comprising the lactic acid bacteria, wherein the lactic acid bacteria is Lactobacillus paracasei KW3110 strain or its mutant;
8. A method for producing a probiotics product which is produced by using lactic acid bacteria, wherein the used lactic acid bacteria is Lactobacillus paracasei KW3110 strain or its mutant;
9. The method for producing a probiotics product according to items 6 to 8, wherein the probiotics product is a beverage or food product;
10. The method for producing a probiotics product according to item 9, wherein the probiotics product is a dairy raw material fermented by using the lactic acid bacteria Lactobacillus paracasei KW3110 strain or its mutant;
11. The method for producing a probiotics product according to items 9 to 10, wherein the probiotics product is a yogurt;
12. The method for producing a probiotics product according to items 6 to 11, wherein the number of the lactic acid bacteria Lactobacillus paracasei KW3110 strain or its mutant in the probiotics product is 5 X 10⁹ or more cells per daily intake;
13. The method for producing a probiotics product according to items 6 to 12, wherein the probiotics product is used for preventing and/or treating allergic diseases;
14. Use of lactic acid bacteria Lactobacillus paracasei KW3110 strain or its mutant in the manufacture of a probiotics product;
15. The use of lactic acid bacteria Lactobacillus paracasei KW3110 strain or its mutant in the manufacture of a probiotics product according to item 14, wherein the probiotics product is a beverage or food product;
16. The use of lactic acid bacteria Lactobacillus paracasei KW3110 strain or its mutant in the manufacture of a probiotics product according to item 15, wherein the probiotics product is a dairy raw material fermented by using the lactic acid bacteria Lactobacillus paracasei KW3110 strain or its mutant;
17. The use of lactic acid bacteria Lactobacillus paracasei KW3110 strain or its mutant in the manufacture of a probiotics product according to items 15 and 16, wherein the probiotics product is a yogurt;
18. The use of lactic acid bacteria Lactobacillus paracasei KW3110 strain or its mutant in the manufacture of a probiotics product according to items 14 to 17, wherein the number of the lactic acid bacteria Lactobacillus paracasei KW3110 strain or its mutant in the probiotics product is 5 X 10⁹ or more cells per daily intake;
19. The use of lactic acid bacteria Lactobacillus paracasei KW3110 strain or its mutant in the manufacture of a probiotics product according to items 14 to 18, wherein the probiotics product is used for preventing and/or treating allergic diseases.

### (Lactic acid bacteria as active ingredients) (reference example)

The lactic acid bacteria with high antiallergic activity being the active ingredients are the lactic acid bacteria showing equal or 60% or more of interleukin 12 production level compared to when *L. paracasei* KW3110 strain is used, and showing less than 50% of interleukin 4 production level of a control wherein the lactic acid bacteria are not added, in case lymphocytes derived from mouse spleen sensitized with ovalbumin (hereinafter referred to as "OVA") are suspended in a medium containing OVA, and cultured by adding the lactic acid bacteria to be tested. More preferable active ingredients of the present invention are the lactic acid bacteria among the lactic acid bacteria mentioned above showing equal or 75% or more of interleukin 12 production level compared with when *L. paracasei* KW3110 strain is used, in case lymphocytes derived from mouse spleen sensitized with OVA are suspended in a medium containing OVA, and cultured by adding the lactic acid bacteria to be tested. Most preferable active ingredients are the lactic acid bacteria among the lactic acid bacteria mentioned above showing less than 40% of interleukin 4 production level compared with when control wherein the lactic acid bacteria are not added is used, in case lymphocytes derived from mouse spleen sensitized with OVA are suspended in a medium containing OVA, and cultured by adding the lactic acid bacteria to be tested.

In one reference aspect of the lactic acid bacteria with high antiallergic activity are the lactic acid bacteria showing equal or 80% or more of interleukin 12 production level compared with when *L. paracasei* KW3110 strain is used as the lactic acid bacteria to be tested, and showing less than 30% of interleukin 4 production level compared with control wherein the lactic acid bacteria are not added, in case lymphocytes derived from mouse spleen sensitized with OVA are suspended in a medium containing ovalbumin (hereinafter referred to as "OVA"), and cultured by adding the lactic acid bacteria to be tested.

To obtain the lactic acid bacteria with high antiallergic activity, the method for obtaining the lactic acid bacteria with high antiallergic activity by separating the lactic acid bacteria showing equal Th1-inducing cytokine producing level compared to when *Lactobacillus paracasei* KW 3110 strain is used as the lactic acid bacteria to be tested, or showing 60% or more of Th1-inducing cytokine producing level compared to when *L. paracasei* KW3110 strain is used, and showing less than 50% of interleukin 4 production level of a control wherein the lactic acid bacteria are not added, in case lymphocytes derived from mouse spleen sensitized with allergen are suspended in a medium containing allergen, and cultured by adding the lactic acid bacteria to be tested. In other words, it can be obtained by using strain of the lactic acid bacteria publicly known or strain of the lactic acid bacteria newly separated, suspending lymphocytes derived from mouse spleen sensitized with OVA in a medium containing OVA and cultured by adding the lactic acid bacteria, and generally by separating the lactic acid bacteria showing equal interleukin 12 production level compared with when *L. paracasei* KW3110 strain are used as the lactic acid bacteria to be tested or showing 60% or more interleukin 12 production level compared with when *L. paracasei* KW3110 strain are used, and showing less than 50% of interleukin 4 production level of control wherein the lactic acid bacteria are not added. As it is described above, the lactic acid bacteria used as active ingredients can be selected and obtained easily by a person skilled in the art, from various lactic acid bacteria publicly known or newly separated with the use of the index of the present invention as a standard, by conducting experiments to estimate antiallergic activity *in vitro.* The details will be disclosed specifically in Example 1.

As for the lactic acid bacteria with high antiallergic activity used as active ingredients in the present invention, *L. paracasei* KW3110 strain, can be obtained from Japan Dairy Technical Association as *L. casei* L14 strain. Meanwhile, though there is a statement of Japan Dairy Technical Association that L14 strain is *L. casei,* when the present inventors have analyzed by using RFLP (Restriction Flagment Length Polymorphism) and AFLP (Amplified Flagment Length Polymorphism) by using RiboPrinter (QUALICON), the strain was determined to be *L. paracasei,* therefore it is stated as *L. paracasei* in the present invention. *L. paracasei* KW3110 used as active ingredients of the antiallergic composition can be obtained from Japan Dairy Technical Association as described in the above, and moreover, it is deposited as FERM BP-08634 at the National Institute of Advanced Industrial Science and Technology, International Patent Organism Depositary, which is an International Depositary Authority, according to Budapest Treaty on the deposit of microorganism for patent procedure.

Moreover, *L. plantarum* KW4110 strain and *L. paracasei* KW3926 strain used as active ingredients of a reference example can be obtained from JCM (Japan Collection of Microorganisms, RIKEN) as *L. plantarum* JCM1149 strain and *L. paracasei* JCM8132 strain, respectively. *L. paracasei* KW3925 strain can be obtained from NRIC (Tokyo University of Agriculture) as *L. paracasei* NRIC1917 strain.

When selecting the lactic acid bacteria used as active ingredients, it is also possible to select the lactic acid bacteria showing also functions of probiotics that are conventionally known, such as function regulating intestines, function lowering cholesterol, or hypotensive function shown in many lactic acid bacteria, besides antiallergic function. Further, when administering orally the lactic acid bacteria that are active ingredients of the present invention, as it is more effective that the lactic acid bacteria remain in the intestinal tract to fully exert their effect, a more effective lactic acid bacteria can be obtained by subjecting the lactic acid bacteria selected and obtained with the standard mentioned above to an adhesion test to cell strain Caco-2 cell strain derived from human intestinal tract, *in vitro,* to select strain having higher cell adhesion ability. *L. paracasei* KW3110 strain that is the active ingredient of the present invention is a lactic acid bacterium being resistant to digestive juice, highly adhesive to intestinal tract, which shows an excellent effect above mentioned when administered orally as antiallergic agent.

The lactic acid bacteria being the active ingredients of the present invention are used as active ingredients of the probiotics product of the present invention, by cultured and proliferated appropriately in a medium for culturing the lactic acid bacteria known to a skilled person such as M.R.S. (de Man, Rogosa, Sharpe) medium and the like, powdered by lyophilizing or by spray-drying with a spray according to need, as viable cells or being sterilized. Furthermore, for process management, the antiallergic activity of the lactic acid bacteria strain obtained can be measured according to need. In other words, as for a method for measuring the antiallergic activity, it is possible to use the method for measuring the antiallergic activity of the lactic acid bacteria wherein the level of Th1-inducing cytokine and/or Th2-inducing cytokine generated by suspending lymphocytes derived from animal spleen sensitized with allergen in a medium containing the allergen, and cultured by adding the lactic acid bacteria to be tested, is measured.

In the present invention, it is possible to mutate the lactic acid bacteria with high antiallergic activity obtained with the above method, to prepare mutant strain with high antiallergic activity and the like and make use of these. As for a means to raise mutation, it can be carried out by a publicly known mutating means such as UV and the like. For example, KW3110 strain being the lactic acid bacteria with high antiallergic activity can be mutated with mutating means, and by using a method for estimating antiallergic activity of the lactic acid bacteria described in the present specification, it is possible to obtain mutant strain with a preferable characteristic, wherein antiallergic activity is increased or other characteristics are mutated and make use of these.

### (Selection of derivative strains)

In the present invention, it is possible to select strain with different characteristics occurred from the lactic acid bacteria strain obtained in the present invention as derivative strain and to make use of these. The method for selecting the microbial strain can be explained as follows by taking KW3110 as an example: KW3110 strain is cultured by a static culture until it reaches a prescribed number of strains, with the use of a medium such as MRS medium, at a prescribed temperature; diluting the culture solution in a fresh MRS medium; suspending 10% of the microbial suspension to PBS (tablet from Dainippoin Pharmaceutical dissolved at a designated concentration) wherein pH of PBS was adjusted to 3.0 with hydrochloric acid; and incubating at 37°C for 3 hours. The microbial suspension treated with acid is diluted with PBS, poured in MRS plate medium, to form colonies. The colonies formed are selected by taking the color tone and the like as index, and further, one strain is selected among these colonies selected based on the color tone, and cultured in a MRS medium for 48 hours. The antiallergic activity of the culture is measured with the method for measuring the antiallergic activity of the present invention (method of Example 2), and the antiallergic activity of each colony is determined. By using this method, the microbial strains with high antiallergic activity are selected and obtained from the strains tested. In the examples of the present invention, strains separated as derivative strain of KW3110 was named No. 90 strain, and are deposited at the National Institute of Advanced Industrial Science and Technology, International Patent Organism Depositary, which is an International Depositary Authority as FERM BP-08635, according to Budapest Treaty on the deposit of microorganism for patent procedure.

### (Preparation of antibody)

To control quality of probiotics products comprising the lactic acid bacteria with high antiallergic activity, or to control the process of manufacturing such products of the present invention, it is necessary to isolate and detect the lactic acid bacteria with high antiallergic activity of the present invention, and to identify the lactic acid bacteria or to examine its content. It is possible to prepare antibody targeting isolation and detection of the lactic acid bacteria, and to make use of it. As an antibody that binds specifically to the lactic acid bacteria with high allergic activity of the present invention, antibodies specific to immunity such as monoclonal antibodies, polyclonal antibodies and the like can be exemplified concretely, and these can be prepared by a common procedure, by using the lactic acid bacteria above mentioned as antigen. However, monoclonal antibodies are more preferable from the point of view of its specificity. As for the method for producing the monoclonal antibodies or polyclonal antibodies, they can be produced with a method already publicly known. For example, as for the method for producing the antibodies, it can be referred to Koehler, G.; C. Milstein, Nature: 256, 495-497(1975); Davis, B. ,R. Dulbecco, H. Eisen,H. Ginsberg; W. Wood, Principles of Microbiology and Immunology vol. 2; Harper & Row, New York, 1973. In other words, the monoclonal antibody can be generated for example from hybridoma cells obtained by immunizing a mouse with the lactic acid bacteria of the present invention and then by fusing splenic cells of the mouse with mouse myeloma cells. To detect the lactic acid bacteria of the present invention by using antibodies binding specifically to the lactic acid bacteria of the present invention, it can be performed with the immunological detecting method by using antibodies publicly known. As for the immunological assays, RIA assay, ELISA method, fluorescent antibody method and the like can be exemplified.

### (Antiallergic activity of the lactic acid bacteria as the active ingredients of the present invention)

When IgE antibodies are generated in response to antigenic stimulation, the IgE antibodies bind to Fc receptors on the surface of the mast cells in the tissues or on the surface of basophils in blood, antigens are then recognized by IgE antibodies bond on the surface of mast cells or on the basophils surface upon the secondary antigenic stimulation (re-invasion of allergen), and crosslinking are formed between the IgE antibodies, and when mast cells or basophils release vast amounts of chemical mediators with this stimulation as a trigger, then various symptoms of allergy appear.

The lactic acid bacteria of the present invention induce strongly interleukin 12 (IL-12) production being the index of Th1 immunity, and at the same time, suppress strongly the interleukin 4 (IL-4) production being the index of Th2 immunity, in an *in vitro* system using mouse lymphocytes. Therefore, the lactic acid bacteria of the present invention have effects for treating/preventing allergy based on the acting mechanism that the production of IgE antibody is suppressed by enhancing Th1 immunity and suppressing Th2 immunity.

Thus, the antiallergic composition of the present invention become particularly effective to environmental allergy such as hay fever, atopic dermatitis, bronchial asthma, chronic allergic rhinitis/bronchial asthma and the like. Moreover, the lactic acid bacteria of the present invention can also comprise the function of probiotics that are conventionally known, such as function regulating intestines, function lowering cholesterol, hypotensive function shown in many lactic acid bacteria, besides antiallergic function mentioned above. It is confirmed that KW3110 strain that is the lactic acid bacteria being the active ingredients in the present invention are excellent not only for strong antiallergic activity and immune-stimulating activity, but also for its adhesiveness to intestinal tract cells, resistance to gastric acid/bile acid, and moreover, also from the point of view of probiotics mentioned above, its effect is expected.

### (Administration of a reference composition)

The above reference compositon can be used as antiallergic agent after formulation by mixing the active ingredients with carrier, excipient, binding agent, diluent and the like that are physiologically acceptable. The antiallergic agents can be administered orally or parenterally. As for oral agents, granule, powder medicine, tablets (including sugar-coated tablet), pill, capsule, syrup, emulsion and suspending agent can be exemplified. As for non-oral agents, injection (for example subcutaneous injection, intravenous injection, intramuscular injection, intraperitoneal injection), infusion, external medicine (for example, transnasally administered agents, percutaneous agents, ointments), suppository (for example rectal suppository, vaginal agent) can be exemplified. These formulations can be formulated according to a conventional method with excipients, additives pharmaceuticallyacceptable. In the meantime, for formulation, so that the antiallergic composition can exert its function appropriately at the right time and effectively *in vivo,* it is preferable to control the time of initiation of elusion, to add the function as agents masking bitterness, or to enhance the stability to oxygen or humidity, for example, as coated materials coated with coating agents having yeast cell wall described in Patent No. 3349677 as main ingredients, or in the form of capsules obtained by capsulizing in soft or hard capsules according to a common procedure, in order to be preferably used in the fields of medicine, health foods and the like. As for excipients or additives pharmaceutically acceptable, carrier, binding agent, flavor, buffer agent, thickening agent, coloring agent, stabilizer, emulsifier, dispersant, suspending agent, preservative and the like can be exemplified. As for carrier pharmaceutically acceptable, magnesium carbonate, magnesium stearate, talc, sugar, lactose, pectin, dextrin, starch, gelatin, tragacanth, methylcellulose, sodium calboxymethylcellulose, low melting wax, cacao butter and the like can be exemplified.

These formulations can be prepared for example as follows. In other words, oral agents can be molded by compressing, by adding for example excipient (for example, lactose, sucrose, starch, mannitol), disintegrator (for example, calcium carbonate, calboxymethylcellulosecalcium), binding agent (for example α-starch, gum alabic, calboxymethylcellulose, polyvinylpyrrolidone, hydroxypropyl cellulose) or lubricant (for example talc, magnesium stearate, polyethylene glycol 6000), then by coating according to need, by a publicly known method for the purpose of masking the taste and keeping enteric property and sustained action. As for coating agents, for example, ethyl cellulose, hydroxymethylcellulose, polyoxyethylene glycol, cellulose acetate phtalate, hydroxypropylmethylcellulose phtalate and eudragit (Rhom, Germany; methacrylate/acrylate copolymer) and the like can be used.

Injections can be prepared by dissolving, suspending or emulsifying the active ingredients with dispersant (e.g. Tween 80 (Atlas Powder, U. S. A), HCO 60 (Nikko Chemicals), polyethylene glycol, carboxymethylcellulose, sodium alginate and the like), perservative (for example, methyl paraben, propyl paraben, benzyl alcohol, chlorobutanol, phenol), isotonization agent (for example, sodium chloride, glycerin, sorbitol, glucose, invert sugar) and the like to aqueous solvent (for example, distilled water, physiological saline, Ringer solution and the like) or oleaginous solvent (vegetable oil such as olive oil, sesame oil, cotton seed oil, corn oil and the like, propylene glycol), and the like. At that time, additives such as disintegration adjuvant (for example sodium salicylate, sodium acetate), stabilizer (for example human serum albumin), soothing agent (for example, benzalkonium chloride, hydrochloric procaine) and the like can be added if desired.

External agents can be prepared by making the active ingredients to a composition in a solid, semisolid or liquid form. For example, the composition in a solid form mentioned above can be prepared by itself or by adding and mixing excipients (for example lactose, mannitol, starch, microcrystalline cellulose, sucrose), thickening agents (for example, natural gums, cellulose derivative, acrylic acid polymer) and the like to make in a powder form. The composition in a liquid form mentioned above can be prepared almost with the same manner as the injection. As for the compositions in a semisolid form, aqueous or oleaginous gel agents or ointments are preferable. Moreover, all of these compositions can comprise pH controller (for example, carbonic acid, phosphoric acid, citric acid, hydrochloric acid, sodium hydroxide), perservative (for example, paraoxy benzoic acid esters, chlorobutanol, benzalkonium chloride) and the like. Suppositories can be formulated by making the active ingredients to an aqueous or oleaginous composition in a solid, semisolid or liquid form. As for oleaginous base used for the compositions, glyceride of higher fatty acid (for example, cacao oil, witepsols (Dynamit Nobel), medium fatty acid (for example, Migliores (Dynamit Nobel)) or vegetable oils (for example, sesame oil, soybean oil, cotton seed oil) can be exemplified. As for aqueous base, polyethylene glycols, propylene glycol can be exemplified. Moreover, as for aqueous gel base, natural gums, cellulose derivative, vinyl polymer, acrylic acid polymer can be exemplified.

### (Use by compounding into foods or drinks)

The probiotics product of the present invention is used as foods or drinks with antiallergic function by compounding into foods or drinks. To use the probiotics product of the present invention by compounding into foods or drinks, the effective dose of the active ingredients is added and compound during the stage of manufacturing law material or after the product is manufactured and the like. Here, the term "effective dose of the active ingredients" relates to the content wherein the active ingredients are ingested within the following range, when the amount generally consumed for each food and drink are ingested.

In other words, as for the dosage or intake of the effective dose of the active ingredients of the present invention to foods or drinks, it can be determined depending on the recipient, the age and body weight of the recipient, symptoms, administered time, dosage form, administering method, the combination of agents and the like. For example, when the active ingredients of the present invention are administered as medicine orally, it can be administered 1 - 3 times per day within the range of: 0.1-100 mg/kg body weight (preferably 1-10 mg/kg body weight) when administered orally, and 0.01-10 mg/kg body weight (preferably 0.1-1 mg/kg body weight) when administered parenterally. The agents having other acting mechanisms used by combining with the active ingredients of the present invention can be also determined appropriately by using the dosage used clinically as standard.

When the dosage or intake of the effective dose of the active ingredients of the present invention to foods or drinks are indicated by the number of the lactic acid bacteria, it is preferable that the intake is 5 × 10⁹ or more per day, more preferably 1 x 10¹⁰ or more per day, most preferably 5 x 10¹⁰ or more per day. Therefore, the number of the lactic acid bacteria to be contained per each food is determined according to the amount of foods or drinks generally ingested per day. For example, when ingesting as food (yogurt), the active ingredients of the present invention can be compounded to the food so that the intake will be within 50 - 500 g per day for an adult, and preferably within 60 - 200 g.

In the present invention, the active ingredients of the present invention can be compounded by itself or in a form of formulation described above to foods or drinks. More concretely, the foods or drinks of the present invention can take various form of usage, by compounding the active ingredients of the present invention with base materials appropriately, and prepare as foods or drinks by itself, or by further compounding various proteins, sugars, fats, trace elements, vitamins and the like, or prepared in a liquid, semi-liquid or solid form, or further added or compounded to general foods or drinks, or the like.

As for the field of foods or drinks using the lactic acid bacteria, it is roughly classified into dairy products, meats, breads, beverages and vegetables, based on examples of its utilization, heretofore. By using the lactic acid bacteria with high antiallergic activity of the present invention as the lactic acid bacteria in manufacturing foods or drinks by using these lactic acid bacteria or as a part of it, or as an additive for foods or drinks manufactured, it is possible to add antiallergic function to the foods or drinks.

Further, it is preferable that probiotics foods or drinks with high antiallergic function of the present invention are produced in a form of foods or drinks filled in a sealed container to prevent contamination of other microorganisms or foreign compounds, to maintain the quality of the content.

The foods of the present invention can be prepared as health food, functional food, specified health food, or patient food wherein antiallergic function is added. Moreover, it is not particularly limited to form of food, and it can be in a form of beverage wherein antiallergic function is added. As the active ingredients of the present invention have antiallergic activity, it is possible to provide foods that are possible to ingest continuously and having function of preventing the development of allergy or treating allergy, by compounding the active ingredients of the present invention to foods ingested daily or health foods or functional foods and the like ingested as supplements. By using the lactic acid bacteria with high antiallergic activity of the present invention by compounding in foods or drinks that do not contain ingredients inducing allergy in the foods or drinks, such as tea drinks, health drinks or tablets, it is possible to provide probiotics foods or drinks with antiallergic function completely blocked from allergic problems. Further, in the present invention, by replacing whole or a part of the lactic acid bacteria of beverages or foods produced using the lactic acid bacteria or comprising the lactic acid bacteria with the lactic acid bacteria with high antiallergic activity by the present invention, it is possible to make a probiotics beverage or food with high antiallergic function.

### (Use in formulation form of foods or drinks)

In the present invention, as for health foods and functional foods compounding the lactic acid bacteria with high antiallergical activity of the present invention, various products can be exemplified, and as for the production of these health foods and functional foods, besides food materials and food additives generally used, it can be used in formulation form of foods or drinks using adjuvants such as excipients, extender, binding agent, disintegrator, lubricant, dispersant, preservative, wetting agent, solving adjuvant, antiseptic, stabilizer, capsule and the like. Examples of the adjuvants that are possible to compound include: lactose, fructose, glucose, starch, gelatin, magnesium carbonate, synthetic magnesium silicate, talc, magnesium stearate, calcium carbonate, methylcellulose, carboxymethylcellulose, or salt thereof, gum alabic, polyethylene glycol, syrup, vaserine, glycerin, ethanol, propylene glycol, citric acid, sodium chloride, sodiumsulfite, sodium phosphate, pullulan, carrageenan, dextrin, reduced palatinose, sorbitol, xylitol, stevia, artificial sweetener, citric acid, ascorbic acid, acidulant, sodium bicarbonate, sucrose ester, vegetable hydrogeneted oil, potassium chloride, safflower oil, bees wax, soybean lecithin, flavor and the like. As for the production of such health foods and functional foods, it can be referred to reference books on drug formulation, for example "Practical guide of Japanese Pharmacopoeia (General rule on formulation)" (Hirokawa Shoten) and the like.

In the present invention, as for forms particularly suitable for health foods and functional foods, form of tablet, capsule, granule, powder medicine, suspension, emulsion can be exemplified, and from the point of view of the object that the present invention is aiming to solve, it is preferable that health foods and functional foods compounding the lactic acid bacteria with high allergic activity of the present invention are combining raw materials that do not comprise raw material specific to allergy.

Examples of methods for producing health foods and functional foods in the form of tablets include a producingmethod wherein the mixture compounding the lactic acid bacteria with high antiallergic activity of the present invention is compressed to a certain form, or a method wherein the mixture being wet with solvent such as water or alcohol is formed in a certain form or poured into a certain mold. Examples of methods for producing health foods and functional foods in the form of capsules include a method of filling capsules wherein the formulation compounding the lactic acid bacteria with high antiallergic activity of the present invention is filled into capsules in form of liquid, suspension, paste, powder or granule, or a producing method by encapsulating and forming with capsule base materials, such as hard capsules or soft capsules and the like.

### (Compounding to foods)

Moreover, in the present invention, it is possible to prepare foods with antiallergic function by compounding the lactic acid bacteria with high antiallergic activity of the present invention to foods. Examples of these foods or drinks include: cakes such as cream caramel, cookie, cracker, potato chips, biscuit, bread, cake, chocolate, donuts, jelly and the like; Japanese cakes such as rice cracker, faded black, daifuku (rice cake filled with sweet jam paste), been cake, other steamed bean-jam bum, sponge cake, and the like; breads/snacks such as cold dessert (candy and the like), chewing gum and the like; noodles such as wheat noodle, buckwheat noodle, kishimen (flat wheat noodle) and the like; fish cakes such as steamed fish paste, ham, fish meat sausage and the like; meat products such as ham, sausage, hamburger, canned beef and the like; seasonings such as salt, pepper, soybean paste (miso), soybean sauce, sauce, dressing, mayonnaise, ketchup, sweetener, pungent seasonings and the like; grilled foods such as akashiyaki (soft octopus ball), takoyaki (octopus ball), monjayaki (doughy crape-like pancake), okonomiyaki (savory pancake), friednoodles, friedwheatnoodles and the like; dairy products such as cheese, hard type yogurt and the like; various prepared foods such as fermented soybeans, pressed tofu, tofu, yam paste, rice dumpling, pickles, fish boiled in soy sauce, kop-zi, shaomai, croquette, sandwich, pizza, hamburger, salad and the like; various powder (meat products such as beef, pork, chicken and the like; fishery products such as shrimp, scallop, freshwater clam, dried tangle and the like; vegetables/fruits, plants, yeast, algae and the like); powdered solid products of fat/flavoring ingredients (vanilla, citrus, bonito and the like) ; powdered foods or drinks (instant coffee, instant tea, instant milk, instant soup, miso soup and the like) and the like, but it is not limited to these.

Particularly, when adding the active ingredients of the present invention in dairy products, it is possible to produce foods or drinks containing fermented the lactic acid bacteria such as yogurt, by adding the active ingredients of the present invention to dairy raw materials as viable cells to proliferate/ferment bacteria.

### (Compounding to beverages)

As for the composition with high antiallergic activity of the present invention, particularly by using it in form of beverage, it is possible to provide a beverage with antiallergic function that can be ingested everyday continuously, with antiallergic function that becomes effective with the amount possible to ingest continuously.

When compounding the lactic acid bacteria with high antiallergic activity of the present invention to beverage, the content of the lactic acid bacteria can be determined appropriately, but generally the amount to be compound is applied such that the antiallergic activity of the lactic acid bacteria can be effective in an ingestible amount continuously as beverage is applied. When the dosage or intake of the effective dose of the active ingredients of the present invention to foods or drinks are expressed by the number of the lactic acid bacteria, it is preferable that the intake is 5 × 10⁹ or more cells per day, more preferably 1 × 10¹⁰ or more cells per day, most preferably 5 x 10¹⁰ or more cells per day. Therefore, the number of the lactic acid bacteria strain to be contained per each beverage is determined, with the index mentioned above, according to the amount of drinks generally ingested per day. For example, if 100 g of beverage is ingested per day, it is preferable to add 10⁹ or more of bacteria per 100 g of beverage. On the other hand, when it is considered not to damage the flavor or the appearance of the beverage by adding the lactic acid bacteria, 10¹¹ or less cells is preferable. Moreover, 5 x 10¹⁰ or less cells is more preferable. Therefore, as for the concentration of the lactic acid bacteria having high antiallergic function, and being stabilized, having good taste and good storage ability, it is most preferable to be within 10⁹-10¹¹ cells per 100 g of beverage. Meanwhile, as for the relationship between the number of the lactic acid bacteria and the weight of dried bacteria, for example, for *L. paracasei* KW3110 strain, the number of strain 10¹² bacteria correspond to 1 g weight of dried strain.

In the present invention, as for beverage compounding the lactic acid bacteria with high antiallergic activity of the present invention, various beverages can be exemplified, and for manufacturing these, any of saccharide, flavor, juice, food additives and the like used for general beverage formulation can be used. As for manufacturing beverages, it can be referred to existing reference books, for example "Revised new edition: soft drinks" (Kohrin) and the like.

In the present invention, particularly as for beverages suitable to compounding, beverages that do not contain dairy ingredients, in other words, for example nondairy beverage such as soft drinks containing juice or tea drinks are particularly preferable in view of antiallergic activity, storage/stabilization of the products.

Examples of beverages to compound include: alcoholic beverage (whiskey, bourbon, spirit, liqueur, wine, fruit wine, rice wine (sake), Chinese wine, distilled spirit, beer, non-alcohol beer with 1% or less of alcohol proof, law-malt beer, chuhai (carbonated distilled spirit) and the like), or non-alcoholicbeverage (drink type yogurt, juiceof apple, orange, grape, banana, pear, plum, watermelon and the like, vegetable juice of tomato, carrot, celery, cucumber an the like, soft drink, milk, soy milk, coffee, cocoa, various herb tea such as red tea, green tea, barley tea, brown rice tea, natural leaf tea, refined green tea, roasted green tea, oolong tea, curcuma tea, black tea, Rooibos tea, rose tea, chrysanthemum tea, mint tea, jasmine tea and the like, sport drink, mineral water, energy drink and the like).

By exemplifying by categories of beverage, tea drink such as green tea, oolong tea, red tea, barley tea, blended tea, and coffee, beverage containing juice, vegetable juice, sport drink, energy drink can be exemplified.

Moreover, as for the form of the product, the form of beverage in a sealed container used generally as product form of beverages is particularly preferable, and as for the sealed container, any form of can, bottle, PET bottle, paper container can be used. Moreover, there is no particular limitation for the volume, and it can be determined generally by considering the amount that a consumer ingests daily, the number of the lactic acid bacteria to be compound, and the number of bacteria necessary daily.

The present invention will be explained in detail in the following, but the present invention will not be limited by these.

### Example 1

### (Lactic acid bacteria used in the (Reference-) Examples of the present invention)

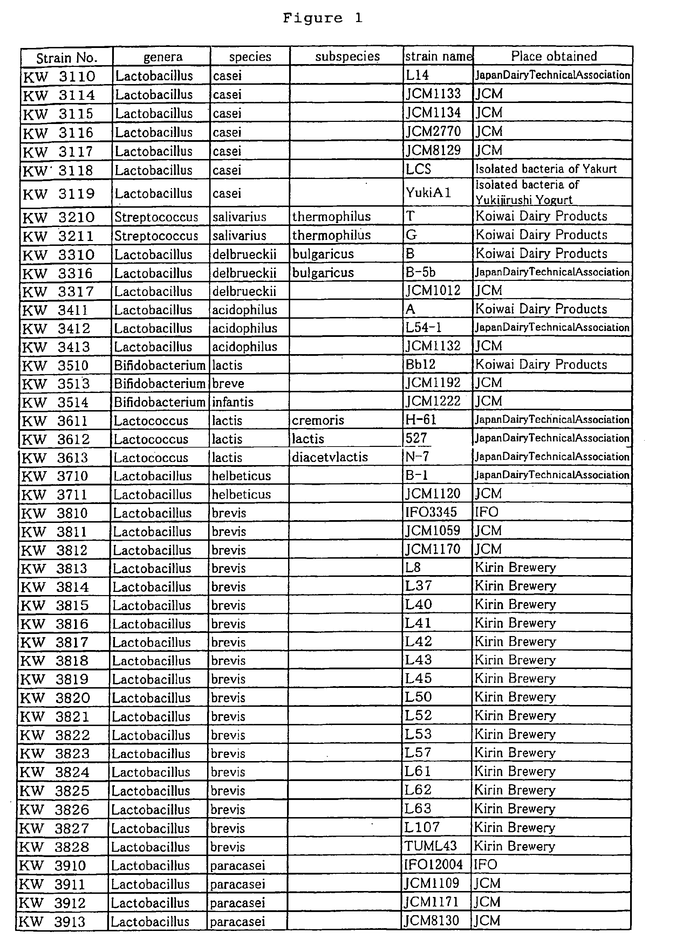

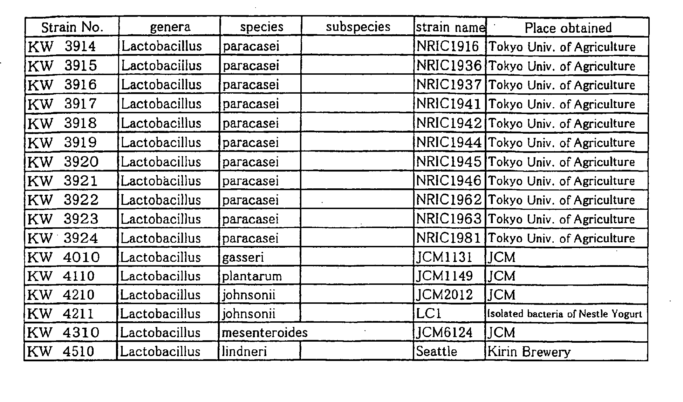

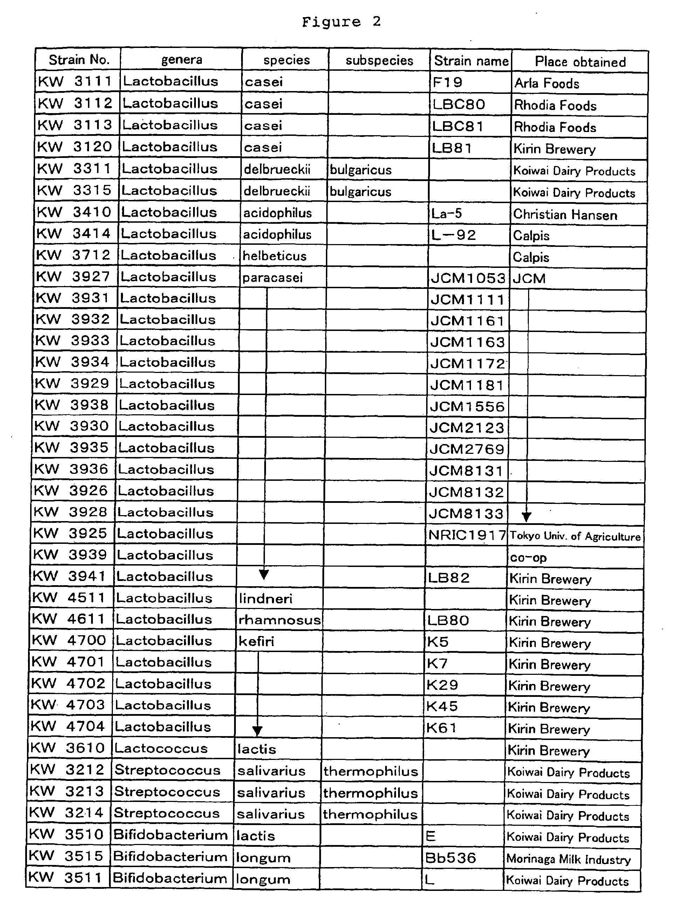

The lactic acid bacteria used in the Examples of the present invention and the place obtained are shown in Fig. 1 (No.1) and Fig. 2 (No.2). In the present specification, all the strains are identified by numbers beginning with KW. As these test strains include strains separated independently, strains used in dairy products commercially available and strains obtained frompublic institutions or the like, unified identification names are used for convenience. The relation between each KW strain and the place obtained are shown in Fig. 1 (No.1) and Fig. 2 (No.2). Meanwhile, for "place obtained", "JCM" is for Japan Collection of Microorganisms, The Institute of Physical and Chemical Research, "IFO" is for former Institute for Fermentation (now changed to National Institute of Technology and Evaluation, Biological Research Center (NBCR)).

### Example 2 (for reference only)

### (Estimation of antiallergic activity of the lactic acid bacteria group in vitro)

### 1. Sample

Each strain of the lactic acid bacteria is cultured in M.R.S. (de Man, Rogosa, Sharpe) medium (OXOID) for 48 hours, washed with sterile water for three times, suspended in sterile water, treated for 30 minutes at 100°C and sterilized. The culture is lyophilized, and suspended in PBS. Refer to Fig. 1 (No.1) and Fig. 2 (No.2) for the lactic acid bacteria used.

### 2. Experimental animals, breeding

Seven to ten weeks-old BALB/c mice (Charles River) were intraperitoneally injected with 1 mg of ovalbumin (OVA) at day 0 and day 6 with 2 mg of Alum being an adjuvant. The animals were dissected on day 13, to isolate spleen and to prepare lymphocytes. Experiments were conducted as n=6.

### 3. Measurement of splenic cells IL-4, IL-12

The measurement of IL-4 and IL-12 were measured by using OptEIA ELISA Set (Becton Dickinson).

### 4. Experimental conditions

Splenic lymphocytes prepared with the method described in the section" 2. Experimental animals, breeding" were suspended in RPMI 1640 (SIGMA) + 10% FCS (Rosche) + 1 mg OVA medium to obtain 2.5 x 10⁶ cells/ml. Then, the lactic acid bacteria to be tested were added at 0.1 or 1 µg/ml. One week later, the supernatant was recovered, IL-12 and IL-4 were measured as Th1 cytokine, Th2 cytokine, respectively.

### 5. Results

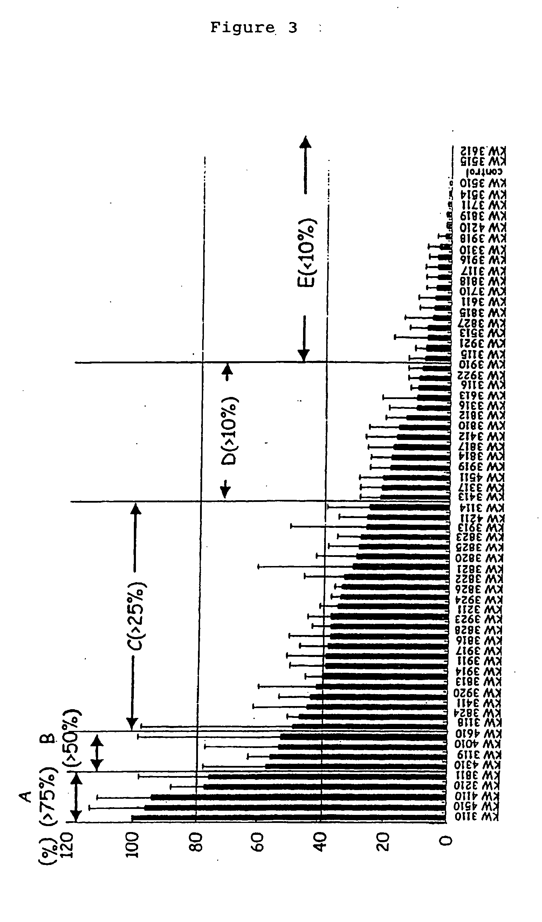

The production levels of Th1 cytokine, IL-12 by each lactic acid bacteria are shown in Fig. 3 and Table 2. The amount added of the lactic acid bacteria is 1 µg/ml. With the difference of strain, various production levels couldbe seen. *L. paracasei* KW3110 strain showed the strongest Th1 induction ability among the approximately 100 strains. All the data are shown as relative ratio to the IL-12 production level by KW3110 strain which was taken as 100%. For convenience, strain showing 75% or more of production level compared with that of KW3110 strain has A rating, strain showing 50% or more to less than 75% of production level has B rating, strain showing 25% or more to less than 50% of production level has C rating, strain showing 10% or more to less than 25% of production level has D rating, strain showing less than 10% of production level has E rating.

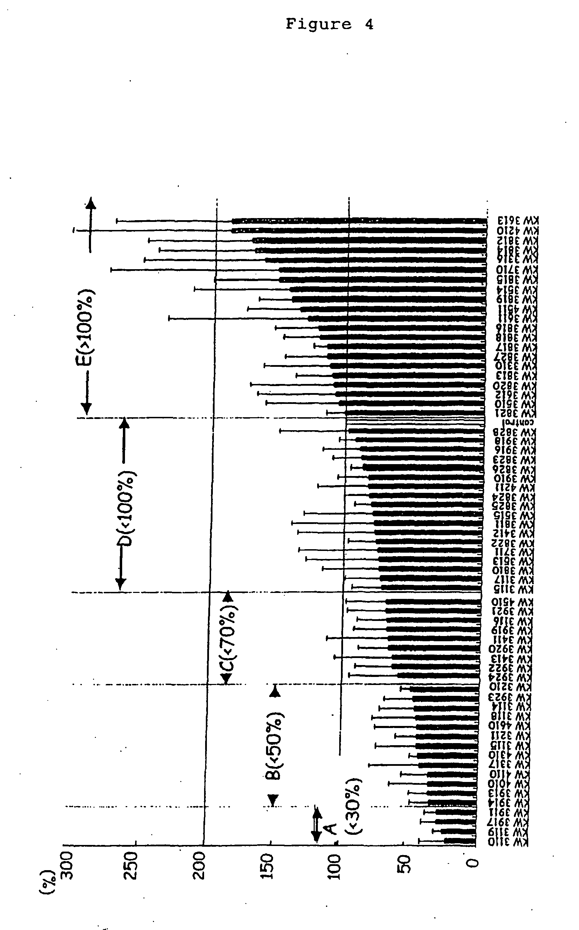

The production levels by each lactic acid bacteria of Th2 cytokine and IL-4 which are the cause of allergy are shown in Fig. 4 and Table 2. The amount added of the lactic acid bacteria is 0.1 µg/ml. With the difference of strain, the suppressing effect showed various levels. *L. paracasei* KW3110 strain showed the most strong Th2 suppressing ability among the approximately 100 strains. All the data are shown as relative ration to the IL-4 production level of control (lactic acid bacteria non-added) is taken as 100%. For convenience, strain showing less than 30% of production level compared with the control has A rating, strain showing 30% or more to 50% of production level has B rating, strain showing 50% or more to less than 70% of production level has C rating, strain showing 70% or more to less than 100% of production level has D rating, strain showing 100% or more of production level has E rating. "Estimation results of antiallergic lactic acid bacteria" in which A-E rating are resumed for each of Th1, Th2 parameters, are shown in Table 1 and Table 2.

**(Table 1)**

| | Th1 | Th2 | | Th1 | Th2 | | Th1 | Th2 | | Th1 | Th2 |
|---|---|---|---|---|---|---|---|---|---|---|---|
| KW3118 | C | B | KW4510 | A | C | KW3910 | D | E | KW3317 | D | B |
| KW3110 | A | A | KW4511 | D | E | KW3914 | C | B | KW3811 | A | D |
| KW4210 | E | E | KW3812 | D | E | KW3916 | E | D | KW3711 | E | D |
| KW3510 | E | E | KW3813 | C | E | KW3917 | C | B | KW4010 | B | B |
| KW3310 | E | E | KW3814 | D | E | KW3918 | E | D | KW3413 | C | C |
| KW3411 | C | C | KW3815 | E | E | KW3919 | D | C | KW4110 | A | B |
| KW3211 | D | C | KW3816 | C | E | KW3920 | C | C | KW3513 | E | D |
| KW3820 | C | F | KW3817 | D | E | KW3921 | E | C | KW3514 | E | E |
| KW4211 | C | D | KW3818 | E | E | KW3922 | E | C | KW4310 | A | B |
| KW4610 | B | C | KW3819 | E | E | KW3923 | C | B | KW3210 | A | C |
| KW3515 | F | D | KW3821 | C | E | KW3924 | C | C | | | |
| KW3810 | D | D | KW3822 | C | D | KW3911 | C | B | | | |
| KW3612 | E | E | KW3823 | C | D | KW3114 | C | B | | | |
| KW3710 | E | E | KW3824 | C | D | KW3115 | E | D | | | |
| KW3611 | E | E | KW3825 | C | D | KW3116 | E | C | | | |
| KW3316 | D | E | KW3826 | C | D | KW3117 | E | D | | | |
| KW3613 | D | E | KW3827 | E | E | KW3913 | C | B | | | |
| KW3412 | D | D | KW3828 | C | D | KW3119 | B | A | | | |

**(Table 2)**

| strain No. | Th 1(% of KW 3110) | rating | Th2 (% of control) | estimation |
|---|---|---|---|---|
| KW3111 | 42.1 | C | 36.3 | B |
| KW3112 | 43.6 | C | 48.8 | B |
| KW3113 | 38.3 | C | 54.8 | C |
| KW3120 | 38.8 | C | 43.9 | B |
| KW3311 | 19.4 | D | 66.9 | C |
| KW3315 | 7.4 | E | 90.4 | D |
| KW3410 | 38.7 | C | 49.2 | B |
| KW3414 | 11.7 | D | 74.6 | D |
| KW3712 | 18 | D | 70.7 | D |
| KW3927 | 53.7 | B | 45.3 | B |
| KW3931 | 19.3 | D | 49 | B |
| KW3932 | 17.9 | D | 57 | C |
| KW3933 | 17.1 | D | 50.1 | C |
| KW3934 | 8.1 | E | 48.7 | B |
| KW3929 | 30.7 | C | 43 | B |
| KW3938 | 1.2 | E | 69.8 | C |
| KW3930 | 19.5 | D | 59.7 | C |
| KW3935 | 5.5 | E | 63.7 | C |
| KW3936 | 5.1 | E | 54 | C |
| KW3926 | 70.1 | B | 30.2 | B |
| KW3928 | 39.2 | C | 59.8 | C |
| KW3925 | 77.1 | A | 45.2 | B |
| KW3939 | 2.3 | E | 59.1 | C |
| KW3941 | 38.3 | C | 50.3 | C |
| KW4511 | 21.4 | D | 134.4 | E |
| KW4611 | 1.3 | E | 55.4 | C |
| KW4700 | 3.6 | E | 109 | E |
| KW4701 | 2.6 | E | 138.2 | E |
| KW4702 | 2.1 | E | 96.5 | D |
| KW4703 | 0 | E | 103.5 | E |
| KW4704 | 2.3 | E | 94.3 | D |
| KW3610 | 1 | E | 104 | E |
| KW3212 | 19 | D | 67.1 | C |
| KW3213 | 65.3 | B | 54.8 | C |
| KW3214 | 37.2 | C | 49.5 | B |
| KW3510 | 0.5 | E | 105.5 | E |
| KW3515 | 0 | E | 79.3 | D |
| KW3511 | 0 | E | 80 | D |

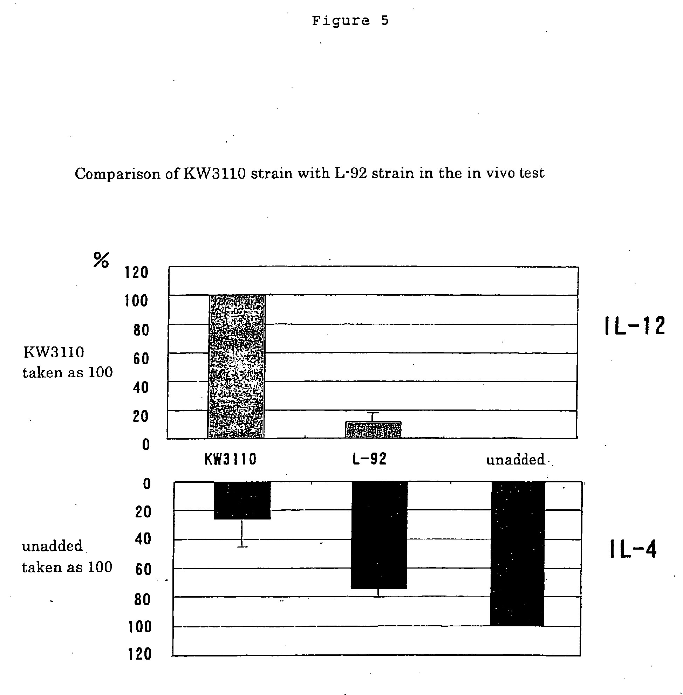

When strains wherein Th1 parameter is 60% or more of KW3110 strain, and Th2 parameter is 50% or less of that of control are determined as antiallergic lactic acid bacteria, relevant strains are the following five strains: KW3110 strain (of the present invention), T strain, NRIC1917strain, JCM8132 and JCM1149 strains. Moreover, when strains showing 75% or more of Th1 induction ability of the strong KW3110 strain are determined as immunostimulating lactic acid bacteria, relevant strains are the following six strains: KW3110 strain, KW4510 strain, JCM1149 strain, T strain, NRIC1917 strain and JCM1059 strain. Thus, immunostimulating composition (medicine, foods or drinks) wherein the lactic acid bacteria, the lactic acid bacteria having equal immunostimulating activity as KW3110 strain, the lactic acid bacteria having equal or more of immunostimulating activity of JCM1059 strain can be manufactured with the method according to the present description. Moreover, *L. acidophilis* L-92 strain known as an antiallergic lactic acid bacteria shows around 11.7% of the IL-12 production level of KW3110 strain, and KW3110 strain shows 26.4% of IL-4 suppressing activity of the control (non-added), whereas L-92 strain shows 74.6% of IL-4 suppressing activity. Thus, for both parameters, it was suggested that *L. acidophilis* L-92 strain is significantly inferior to a strong antiallergic lactic acid bacteria such as KW 3110 strain (Fig. 5). The antiallergic activity of the lactic acid bacteria of the present invention and the lactic acid bacteria strain as the controls was measured and the results are shown in Table 3.

**(Table 3)**

| Srain ID | Genera | Culture collection | Th1 | Th2 |
|---|---|---|---|---|
| KW3110 | *Lactobacillus paracasei* L14 | Japan Dairy Technical Association | 100 | 26.4 |
| KW4110 | *Lactobacillus plantarum* JCM1149 | JCM | 93.9 | 36.7 |
| KW3210 | *Streptococcus salivarius* subsp. Thermophilus T | Koiwai | 77.7 | 49.9 |
| KW3925 | *Lactobacillus paracasei* NRIC1917 | Tokyo Univ. of Agriculture | 77.1 | 45.2 |
| KW3926 | *Lactobacillus paracasei* JCM 8132 | JCM | 70.1 | 30.2 |
| KW4310 | *Lactobacillus mesenteroides* JCM6124 | JCM | 57.4 | 43.4 |
| KW3119 | *Lactobacillus casei* Y A1 | Yukijirushi | 56 | 27.5 |
| KW3927 | *Lactobacillus paracasei* JCM1053 | JCM | 53.7 | 45.3 |
| KW4010 | *Lactobacillus gasseri* JCM 1131 | JCM | 53.2 | 36.7 |

### Example 3 (for reference only)

### (Selection of derivative strains)

The present example is an example where strains with antiallergic activity derived from the lactic acid bacteria strain KW3110 are selected. KW3110 strain was cultured by a static culture in a MRS medium at 37°C, until OD600 varied from 0.8 to 1.0.

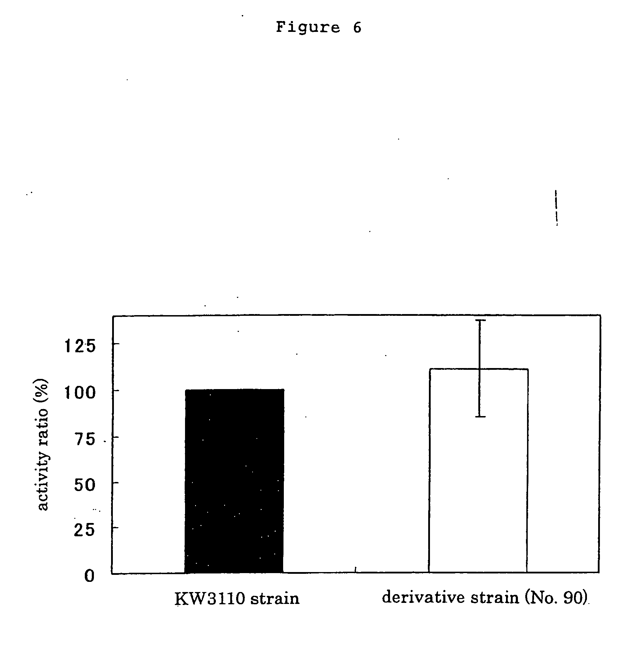

The culture solution was diluted in a fresh MRS medium to obtain OD600 = 0.5, and then 10% of the suspended solution of the strain were suspended in PBS(tablets from DAINIPPON SEIYAKU dissolved at a determined solution), pH of which was adjusted with HCl, incubated at 37°C for 3 hours. The bacterial suspension treated with acid was diluted in PBS, poured on MRS plate medium, and colonies were formed at 37°C. A colony with pale color tone was observed among the colonies formed. This colony was selected and cultured in MRS medium for 48 hours. This strain was named No. 90. The antiallergic activity of No. 90 strain was measured with the method of Example 2. No. 90 strain showed equal IL-12 production ability compared with that of wild-type strain (Fig. 6). Meanwhile, in the figures, "activity ratio (%)" is shown by the IL-12 production ability of the derivative strain (No. 90), when the IL-12 production ability of KW3110 strain was set as 100. The No.90 strain was deposited at National Institute of Advanced Industrial Science and Technology, International Patent Organism Depositary, as FERM BP-08635.

### Example 4 (for reference only)

### (Estimation in vivo of the in vitro high antiallergic activity KW3110 strain)

### 1. Sample administered

*L. paracasei* KW3110 strain was cultured in MRS medium for 48 hours, washed with sterile water for three times, suspended in sterile water and treated at 100°C for 30 minutes to sterilize. The mixture was lyophilized, to prepare mixed feed by mixing it to a standard powdered feed AIN93 (standard composition according to the American Nutrition Research Center), so that a mouse ingests 1 mg per day.

### 2. Experimental animals, breeding

Eight-week old BALB/c mice were used. Group of six BALB/c mice was bred and conditionsed for one week with free intake of CE-2 (JAPAN CLEA) and water. After antigen sensitization at day 0, control groups were bred with powdered feed AIN93 prepared by using refined materials, and KW3110 groups were bred with feeds wherein KW3110 strain was mixed in AIN93.

### 3. Measurement of IgE in blood, IL-4, IL-12 in splenic cells

IgE, IL-4 and IL-12 were measured by using OptEIA ELISA Set (Becton Dickinson).

### 4. Experimental conditions

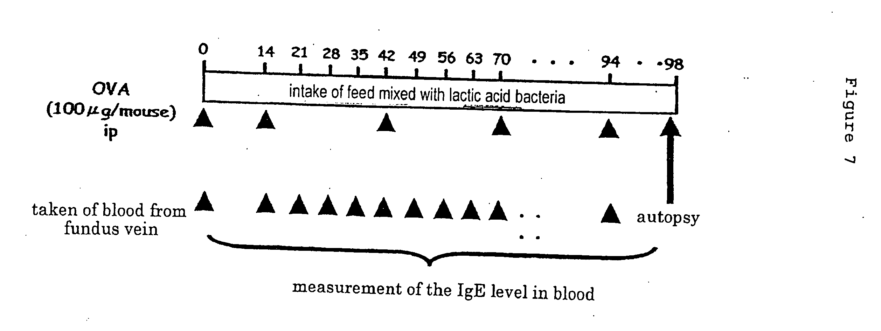

As experimental group, control group (AIN93) and groups wherein KW3110 strain was added toAIN93 at 1 mg/mouse/day (KW3110 group) were set. 4 g of powdered feed pasted with water were administered to both control group and KW3110 group everyday until the termination of the experiment (day 98). Drinking of water was free. Allergic sensitization with ovalbumin (OVA) was performed by injecting intraperitoneally for the total of five times 100µg of OVA and 2mg of Alum being an adjuvant, on day 0, 14, 42, 70 and 94. During this period, blood was collected from fundus vein every week. At the end of experimentation, whole blood was collected and spleen was extracted to prepare lymphocytes (Fig. 7). Splenic lymphocytes were cultured in RPMI1640 (SIGMA) + 10% FCS (Rosche) + 100 µg OVAmedium at 37°C, under 5% Co₂ conditions for one week, and the culture supernatant was collected. IgE level for blood sample, and IL-4 and IL-12 for the splenic lymphocyte culture supernatant sample were measured, respectively.

### 5. Results

The variation of IgE in blood during 98 days of the experimental period is shown in Fig.8. As for IgE in blood on day 48, KW3110 group showed significantly lower IgE level compared with control group, at p < 0.05. IgE levels in blood on day 48 and on day 98 at the time of dissection are shown in Fig. 9 by dot-blot. On day 98 also, IgE in blood for KW3110 group showed significantly lower IgE level compared with control group. IL-12 production level in the splenic cell culture on day 98 of dissection is shown in Fig. 10, and IL-4 production level in Fig. 11, for both control group and KW3110 group. KW3110 group showed significantly high IL-12 production compared with control group. As for IL-4, though there were no significant differences, KW3110 group showed lower tendency against control group. The results mentioned above suggest that by ingesting KW3110 strain, the immune balance in the body shift to Th1, and that allergic status was improved. Further, as it is shown in Fig. 12, on day 30, hair loss around the nose was observed for control group without exception, whereas no hair loss was observed for KW3110 group without exception. At that time, nose scratching behavior was observed frequently for the control group. For KW3110 group, hair loss around the nose or scratching behavior similar to control group was observed around day 60. The observation results of nose scratching behavior suggest that KW3110 groups are actually suppressing allergic symptoms.

### Example 5 (for reference only)

### (Comparison withantiallergiclactic acid bacteriaKW4610strain (L. rhamnosus LGG strain), publicly known)

### 1. Administered sample

KW3110 strain, KW4610 strain were cultured in MRS medium for 48 hours, washed three times with sterile water, suspended in sterile water, treated at 100°C for 30 minutes and sterilized. The mixture was lyophilized, and mixed feed was prepared by pasting the mixture to standard mixed feed AIN93 (standard composition according to the American Nutrition Research Center) so that a mouse ingests 1 or 10 mg per day.

### 2. Experimental animals, breeding

Eight-week old BALB/c mice were used. Group of six BALB/c mice was bred and conditioned for one week with free intake of CE-2 (JAPAN CLEA) and water. From 21 days before antigen sensitization, mixed feed of KW3110 strain and KW4610 strain or AIN93 powdered feed was given to the mice.

### 3. Measurement of IgE in blood

IgE was measured by using OptEIA ELISA Set (Becton Dickinson).

### 4. Experimental conditions

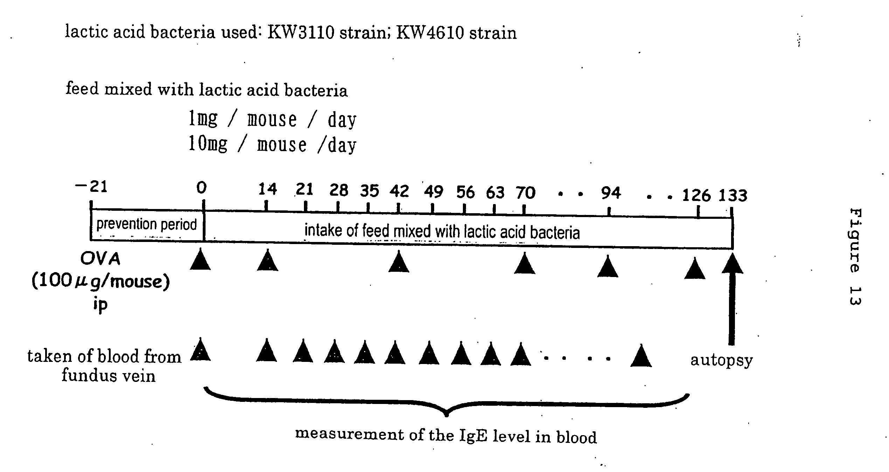

As experimental group, control group (AIN93) and groups wherein KW3110 strain or KW4610 strain was added to AIN93 at 1 or 10 mg/mouse/day (KW3110 group or KW4610 group) were set. 4 g of powdered feed pasted with water were administered to control groups, KW3110 group and KW4610 group everyday until the termination of the experiment (day 133). Drinking of water was free. Allergic sensitization with ovalbumin (OVA) was performed by injecting intraperitoneally for the total of six times 100 µg of OVA and 2 mg of Alum being an adjuvant, on day 0, 14, 42, 70, 98 and 126. During this period, blood was collected from fundus vein every week (Fig. 13). IgE level was measured for blood sample.

### 5. Results

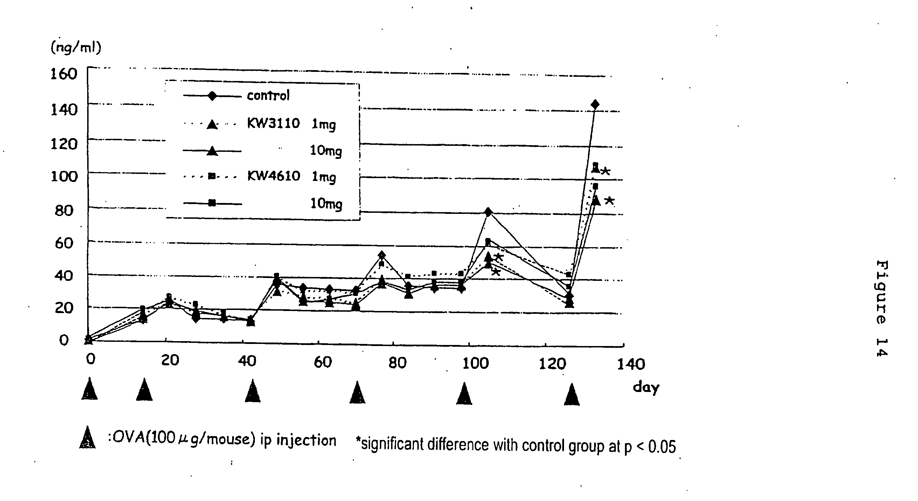

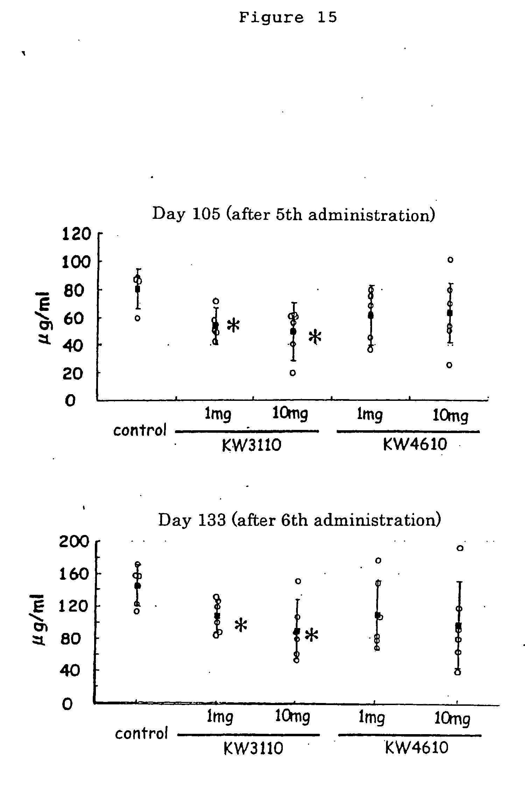

The variation of IgE level in blood during the experimental period is shown in Fig. 14, and IgE level in blood per individual mouse after 5th and 6th injection of OVA is shown in Fig. 15 by dots. With the antigen sensitization by OVA, it was observed that IgE level in blood increased in all the groups. However, a significant decrease of IgE level in blood was observed for KW3110 group compared with control after the 5th administration of OVA. On the other hand, for KW4610 group, no significant decrease of IgE level in blood was observed compared to control, and it was suggested that the antiallergic activity of KW3110 strain was stronger. Furthermore, it was suggested there was a stronger IgE decreasing effect in the 10-mg ingesting group than the 1-mg ingesting group.

### Example 6

### (Resistance to acid and to bile acid of lactic acid bacteria)

It is said that lactic acid bacteria need to be resistant to digestive juice such as gastric acid and bile acid, so that the lactic acid bacteria can reach the intestinal tract alive, and to show probiotic effects. The resistance to acid and resistance to bile acid *in vitro* were measured to investigate whether KW3110 strain is a strain that meet these conditions.

### 1. Methods

The resistance to acid was measured as follows: the lactic acid bacteria were cultured until logarithmic growth anaphase, and an amount of 1/10 of the bacteria being adjusted in PBS (pH 6.5) to be OD600 = 0.5 was added to MRS medium adjusted with hydrochloric acid to be pH 3.0, and incubated at 37°C. 1 hour, 2 hours, 3 hours after the initiation of the incubation, sampling was performed and the viable cell ratio was measured in MRS agar medium.

### 2. Results

The resistance to bile acid was measured as follows: bile acid (OXOID Bile Salts) was added to the MRS liquid medium to the final concentration of 2%, to which the KW3110 strain preculture solution was inoculated by 1%, OD630 was measured by using BIOPLOTTER (Oriental Instruments), and the growth rate was measured.

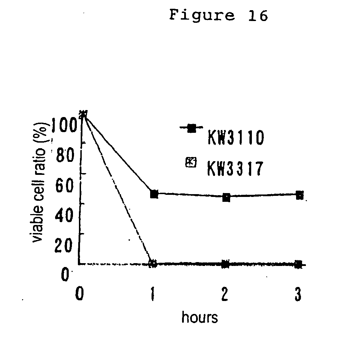

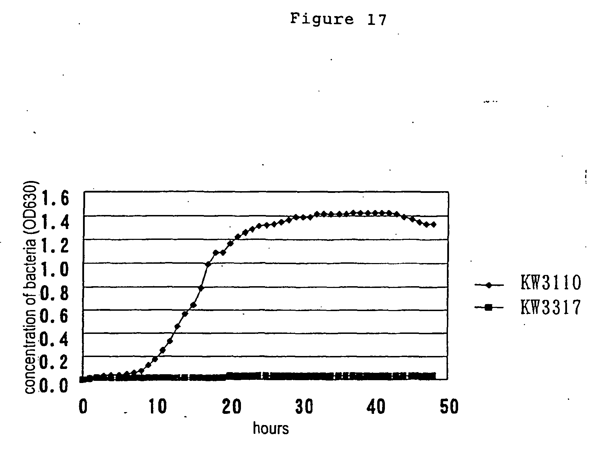

For all the experiments, KW3317 strain being the type strain of *L. delbruckii* (JCM1012 strain) was used as control. As a result, it was suggested that approximately 50% of KW3110 strain were alive for three hours in the acid resistance test (Fig. 16). Moreover, in the bile acid resistance test, KW3110 strain showed sufficient growth also in a medium containing 2% of bile acid, and it was shown to be resistant to bile acid (Fig. 17). On the other hand, it was suggested that KW3117 strain has low resistance to both acid and bile acid.

### Example 7

### (Adhesiveness of the lactic acid bacteria to intestinal tract)

It is necessary that the lactic acid bacteria having reached the intestinal tract alive should remain in the intestinal tract, to show probiotic effects. As an index for the lactic acid bacteria to remain in the intestinal tract and to fully exert their probiotic effects, adhesiveness test to Caco-2 cell from human intestinal tract *in vitro* are used frequently. Therefore, the adhesiveness ability to Caco-2 cells of KW3110 strain was measured.

### 1. Methods

Caco-2 cells were cultured according to the method of Coconnier et al. (Applied and Environmental Microbiology, Vol. 58, p. 2034, 1992). 1.2 x 10⁴ cell/cm² of Caco-2 cells were cultured on a slide glass for about one to two weeks, until being post confluent. KW3110 strain used was cultured in MRS medium for 37°C for two nights and collected, washed once with PBS, adjusted to OD600=1.0 with PBS. The slide glass on which Caco-2 cells were cultured was submerged in 2 ml of KW3110 solution being prepared, contacted at 37°C for 1 hour, under the presence of 10% carbon dioxide, and then the slide glass was washed three times in DMEM (Dulbecco's Modified Eagle Medium) medium. Next, after the slide glass was fixed with methanol, KW3110 strain was stained by Gram staining (Medical Technology, Vol.23, p. 205, 1995). The strain was examined with a microscope, the number of the lactic acid bacteria adherent to Caco-2 cells was count and the mean value per four visual fields was calculated as adherent number. Further, similar investigations were performed for 15 strains of *L. paracasei, L. casei* including KW3913 strain (JCM8130 strain) and KW3115 strain (JCM1134 strain), being the type strain.

### 2. Results

The examination results of adhesive ability to Caco-2 cells of each lactic acid bacteria are shown in Table 4. Ouwehand et al. have reported that the adhesive ability to Caco-2 cells of *L. paracasei, L. casei* was low (Food microbiology and safety, vol. 66, p. 856, 2001). Similarly in the present experiment, strains other than KW3110 strain showed low adhesive ability. However, KW3110 strain showed a significant high adhesive ability to Caco-2 cells among *L. paracasei* and *L. casei* strains, and it was suggested to be a strain wherein excellent probiotic effect can be expected.

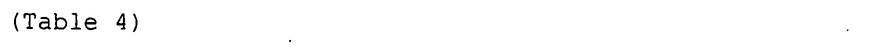

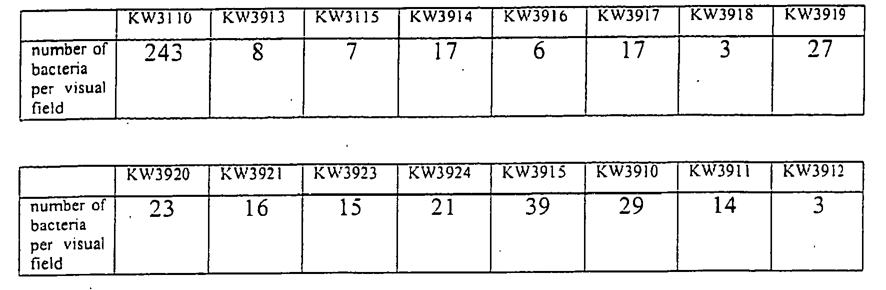

### Example 8 (for reference only)

### (Improvement effect of KW3110 strain for hay fever/airway inflammation by using pollen antigen)

### 1. Methods

### (Type of animal)

Four-week old BDF1 female mice (Charles River) were purchased.

### (Experimental group)

Experiment A: antigen sensitization by nasal drop (model with hay fever)
Control group: group being administered standard feed (AIN-76 base)
KW group: group being administered 1 mg KW/mouse (1 mg KW3110/3 g AIN-76 base)
Experiment B: antigen sensitization by using a nebulizer (model with airway inflammation)
Control group: group being administered standard feed (AIN-76 base)
KW group: group being administered 1 mg KW/mouse (1 mg KW3110/3g AIN-76 base)

### (reagent/instruments)

extract of cedar pollen CP (LSL)
ALUM (Sigma)
ultrasonic nebulizer XE-U12 (OMRON)
cytocentrifuge: Cytospin4 (ThermoBioAnalysis)

### (Methods for estimating)

### Experiment A

BDF-1 mice were conditioned for one week after being purchased, and classified into groups base on the total IgE concentration in blood based on blood plasma obtained by collecting blood from orbit (n=7). After classification into groups, experimental food was initiated to be administered. Further, after three weeks from classification, CP+ALUM solution (CP 10 µg + ALUM 2 mg/mouse) was injected intraperitoneally once a week for three weeks. Two weeks after the third injection of CP+ALUM solution being administered, CP solution (1 mg/ml) was dropped nasally 10 µl each into both noses of the mouse. Further, as a non-stimulated model (Basal), mice being administered with saline for nasal drop were set. Nasal drop was conducted for five days, the number of times of sneeze during five minutes after nasal drop (day 1, 3, 5) and number of times of nose scratching (day 3, 5) were counted.

### Experiment B

BDF-1 mice were conditioned for one week after being purchased, and classified into groups based on the total IgE concentration in blood based on blood plasma obtained by collecting blood from orbit (n=7). After classification into groups, experimental food was initiated to be administered. Further,afterthree weeksfrom classification,CP+ALUMsolution (CP 10 µg + ALUM 2 mg/mouse) was injected intraperitoneally once a week for three weeks. Two weeks after the third injection of CP+ALUM solution, CP solution (40 µg/ml) was nebulized to mice with a nebulizer for 15 minutes in a sealed container. Further, as a non-stimulated model (Basal), mice being nebulized with saline for nasal drop were set. Nebulization was conducted for five days. Further, blood was collected from orbit at the time of CP intraperitoneal injection (day 0), at the initiation of nebulizer (day 35), at the time of dissection (day 40), to measure total IgE concentration in blood. On day 5, bronchoalveolar lavage fluid (BALF) was recovered under anesthesia, number of cells in BALF was counted, cells in BALF were identified, and cytokine in BALF was measured. The BALF was recovered by injecting 2. 1 ml (0.7 ml x three times) of saline with 0.1% BSA into canulated mice airway. After centrifugation, supernatant and cells were recovered, and the number of cells was counted. Furthermore, smear sample was prepared, and cells were identified by Light Giemsa staining.

### 2. Results

### (Experiment A)

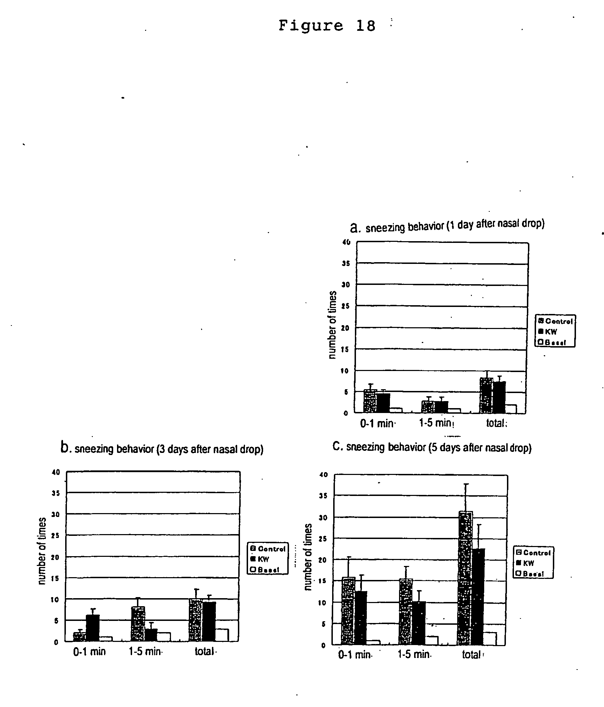

The number of times of sneeze during five minutes after nasal drop was estimated by the sneezing counts in the periods of 0-1 min and 1-5 min after the nasal drop and by the total sneezing counts throughout the period to consider the influence of physical stimulation. As a result, almost no sneeze was observed with nasal drop of saline. However for control group, the frequency of sneeze increased as nasal drop of antigen was repeated. On the other hand, for KW group, it was observed that sneezing had tendency to be suppressed (Fig. 18 a, b, c).

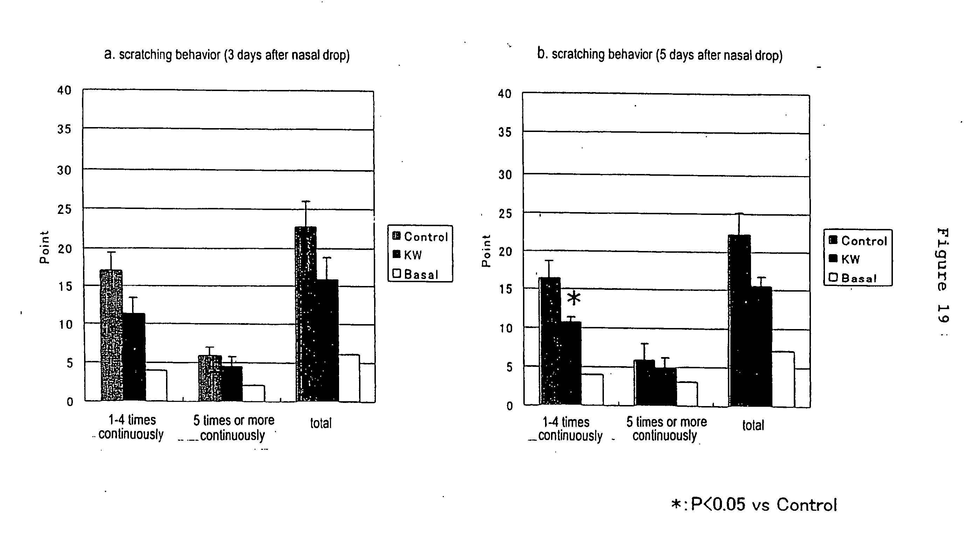

Further, as for the nose scratching behavior, continuous scratching behavior during five minutes after nasal drop was counted as 1 point (total number of points). Moreover, in case of dividing into mild scratching (1-4 times continuous scratching behavior) and severe scratching (5 times or more of continuous scratching), it was estimated by the number of points. Compared with control group, scratching behavior for KW group was suppressed, and a significant suppression was observed for the number of points for mild scratching behaviors on day 5 (Fig. 19 a, b).

### (Experiment B)

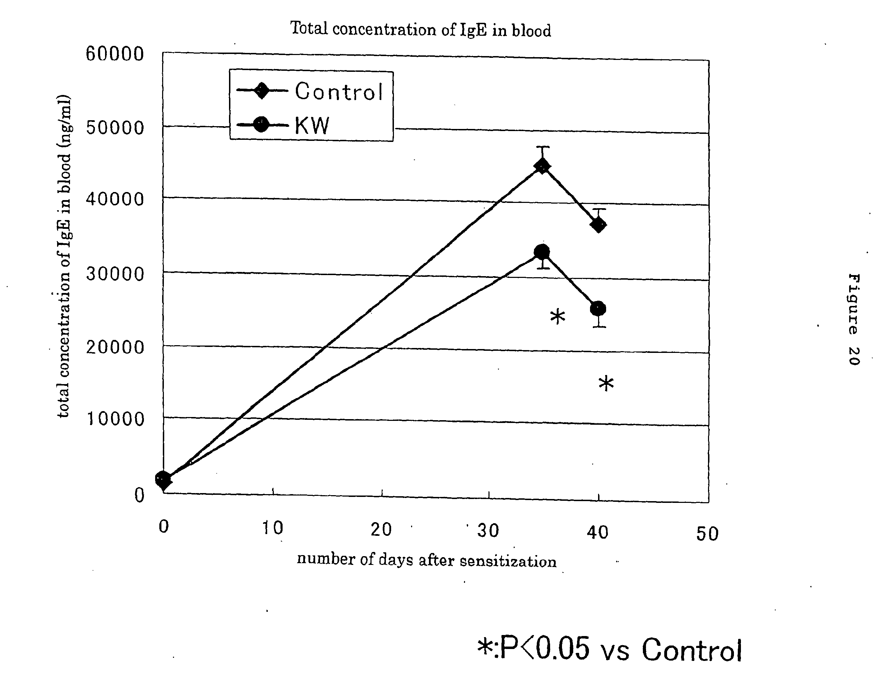

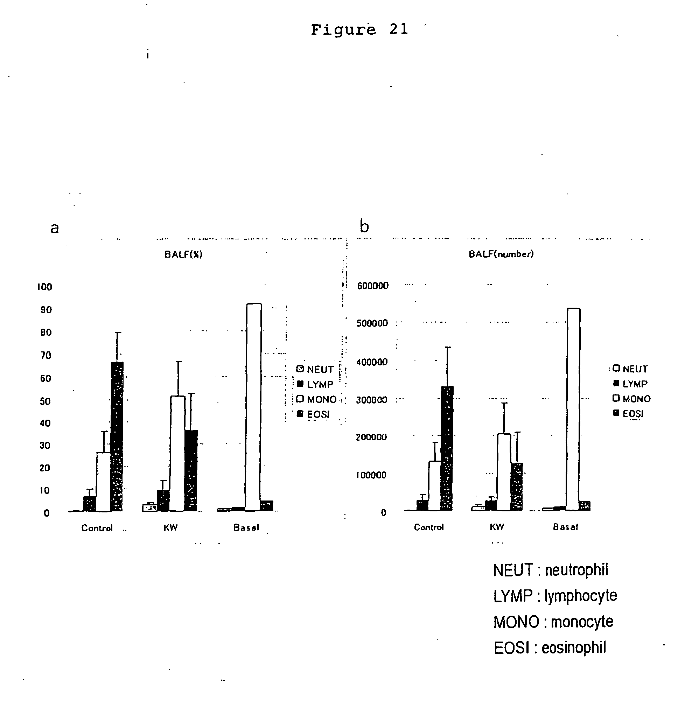

Before nebulizing and at the time of dissection, increase of total IgE concentration in blood for KW group was significantly suppressed compared with control group (Fig. 20). Generally, the types of cells in BALF are for the most of the cases monocytes, mainly macrophages. However, by conducting antigen stimulation, eosinophils cover approximately 65% in BALF for control group, and the monocyte ratio decreases. On the other hand, increase of eosinophil ratio seemed to be suppressed compared with the control group (Fig. 21 a). Moreover, as for the number of eosinophils, a similar tendency that KW group was suppressing topical invasion of eosinophils was observed (Fig. 21b).

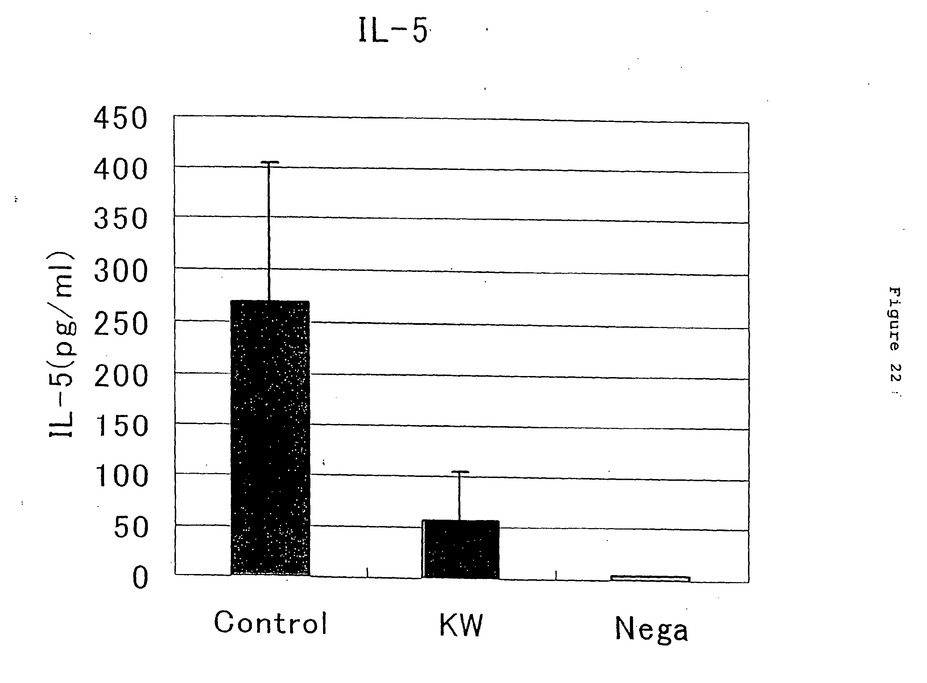

Furthermore, by measuring cytokines in the supernatant of BALF, KW group showed a low level of IL-5 being Th2 cytokine having a role of proliferation/differentiation of eosinophils, compared with the control group (Fig. 22).

From the results mentioned above, it was suggested that there is a possibility that by ingesting KW3110, sneezing behavior and scratching behavior caused by cedar pollen antigen, which are close to clinical symptoms of hay fever among immediate allergy, are suppressed. Further, it was also found that KW3110, suppresses IL-5 production in lung region against airway inflammation caused by exposure to antigen, and further suppress the invasion of eosinophils by suppressing IgE increase. The relation between IL-5 and airway inflammation/eosinophils is described in J. Allergy Clin. Immunol 88(6) 935-942, 1991, suggesting that the administration of KW3110 is effective to allergic asthma, whose main symptoms are airway inflammation.

### Example 9 (for reference only)

### (Improvement effects of KW3110 strain to mouse model having atopic dermatitis)

To investigate improvement effects of KW3110, atopic dermatitis model was used by applying picryl chloride. (reference: OHYO YAKURI (pharmacometrics) 59(6), 123-134, 2000).

### 1. Methods

### (Type of animal)

Five-week old NC/NgaTndCyj male mice (Charles River) were purchased.

### (Experimental group)

Control group: group wherein standard feed (AIN-76 base) was administered for 11 weeks.
KW1 mg-11 wk group: group wherein 1 mg KW3110/3 g AIN-76 base was administered for 11 weeks.
KW10 mg-11 wk group: group wherein 10 mg KW3110/3 g AIN-76 base was administered for 11 weeks.
KW1 mg-8 wk group: group wherein standard feed was administered for 3 weeks, and then 1 mg KW3110/3 g AIN-76 base for 8 weeks.

### (Reagent)

picryl chloride: PCl (TOKYO KASEI KOGYO)
solution for sensitization: 5% PCl solution (ethanol:aceton = 4:1)
ventral part: 100 µl, posterior limb: 25 µl both limbs challenge solution: 0.8% PCl solution (olive oil)
auricle part: 10 µl each for both sides, both ears (total 40 µl), dorsal part: 50 µl

### (Estimation method)

NC/Nga mice were purchased and conditionsed for one week, and were classified based on the total IgE concentration in blood according to serum obtained by collecting blood from orbit (n=10). Three weeks after initiating administrating experimental food upon classification, solution for PC1 sensitization was applied to ventral part being hair shaved, and to both sides of posterior limbs; then, as challenge, one week after, challenge solution was applied to both auricle parts and to both sides, and to dorsal parts being hair shaved. This challenge was conducted every two weeks for 77 times in total. Moreover, to confirm there was no thickening of auricle caused by the solution solvent for challenge, thickening of auricle was measured at 0, 1, 4, 24, 72, 96, 120, 144, 168 hours after application of olive oil, with a dial thickness gage. Further, after the first and third challenge, thickening of auricle was measured similarly at 0, 1, 4, 24, 72, 96, 120, 144, 168 h with a dial thickness gage, to estimate edema. Further, after sensitization, blood was collected from orbit every week, and clinical score was estimated twice a week after sensitization. One week after the 7th and final challenge, mice were sacrificed and serum and auricle tissues were collected. For the serum, total IgE concentration was measured, and for the auricle tissues, hematoxilin eosin staining and Toluidine blue staining were conducted.

### (Clinical score)

Five items, i.e. essential pruritus, reddening/bleeding/erosion, edemo, damage/defect of tissues, forming of scab/desiccation of auricle part; three items, i.e. hair loss, flare/bleeding/erosion, forming of scab/ desiccation of the head, that is eight items in total, were scored as no symptoms (0 point), mild (1 point), moderate (2 points), severe (3 points), and the total was estimated as score.

### (Statistics)

For the statistics, dispersed analysis and Dunnett's multiple comparison test for the control group were conducted, and for each items, it was considered as significantly different when risk rate was 5% or less.

### 2. Results

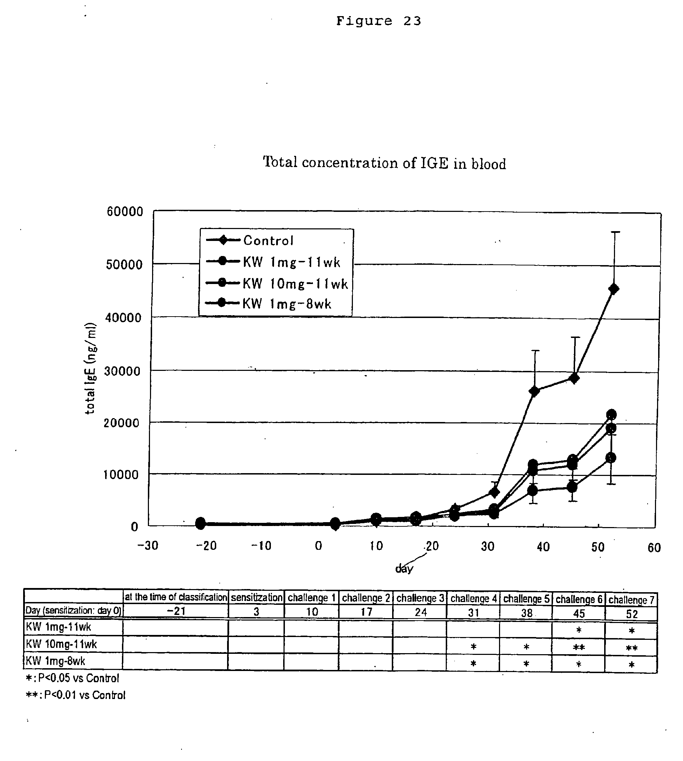

Total IgE concentration in blood for the control group increases significantly from approximately four weeks after PCl challenge. However, for all the groups having ingested KW3110 the increase was suppressed, it was significantly suppressed from the 4th challenge for KW10 mg-11 wk group and KW1 mg-8 wk group and from the 6th challenge for KW1 mg-11 wk group (Fig. 23).

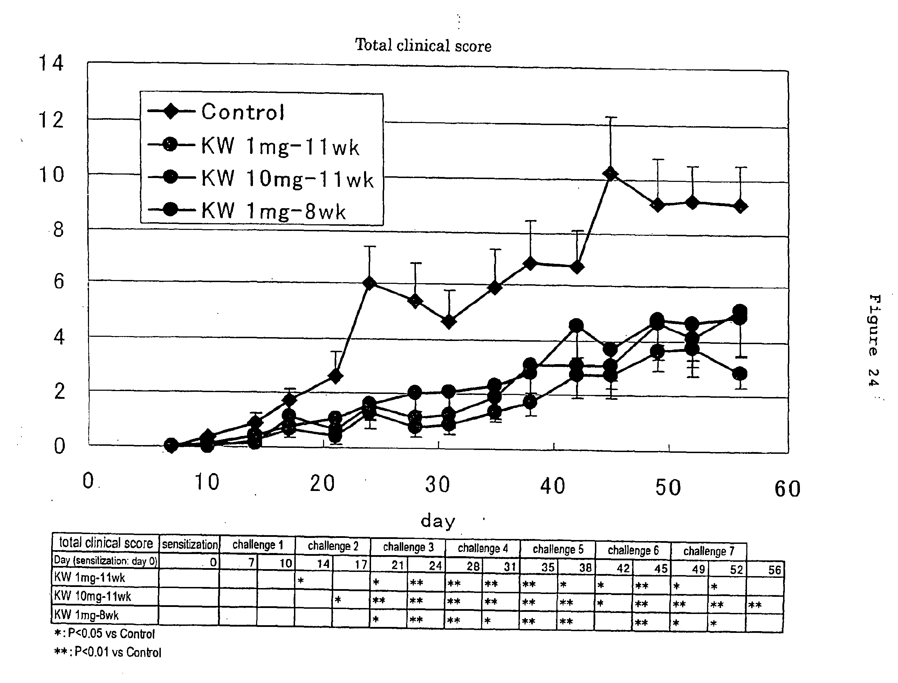

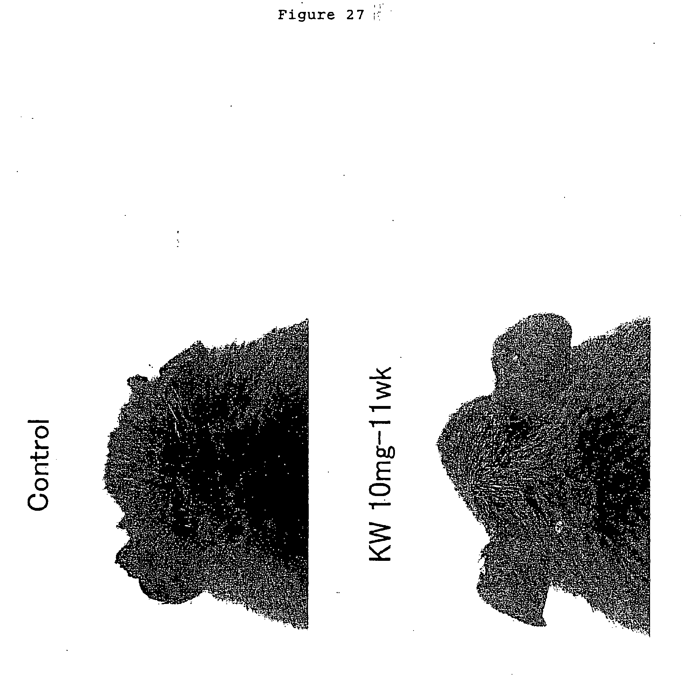

For the clinical score, deterioration of diseases for auricle part and scalp was observed for the control group from early stage, in other words, increase of score was confirmed. On the other hand, the increase was suppressed in all groups having ingested KW3110 strain. The increase was significantly suppressed after the second challenge for KW1 mg - 11 wk group and KW10 mg - 11 wk group, and from just after the third challenge for KW1 mg - 8 wk group. As for the clinical score for only the auricle part, or for only scalp part, significant difference was similarly observed for all groups having ingested KW3110 (Fig. 24, 25, 26). Further, it can also be confirmed from the picture of scalp that erosion/damage of tissues for the auricle part is suppressed for the groups having ingested KW (Fig. 27).

For thickening of auricle caused by PCl challenge, no thickening of auricle was observed by the application of solvent performed during the sensitization stage, but thickening/reddening/edemo of auricle were observed by conducting challenge with PCl. After the first challenge, for the control group, the first phase of thickening that is said to be associated with immediate reaction was observed on 1 and 4 hours after the challenge; the second phase of thickening that is said to be associated with delayed reaction was observed 48 hours after the challenge, and further the third phase of thickening of auricle part that is said to be associated with immediate reaction was observed 120-144 hours after the challenge.

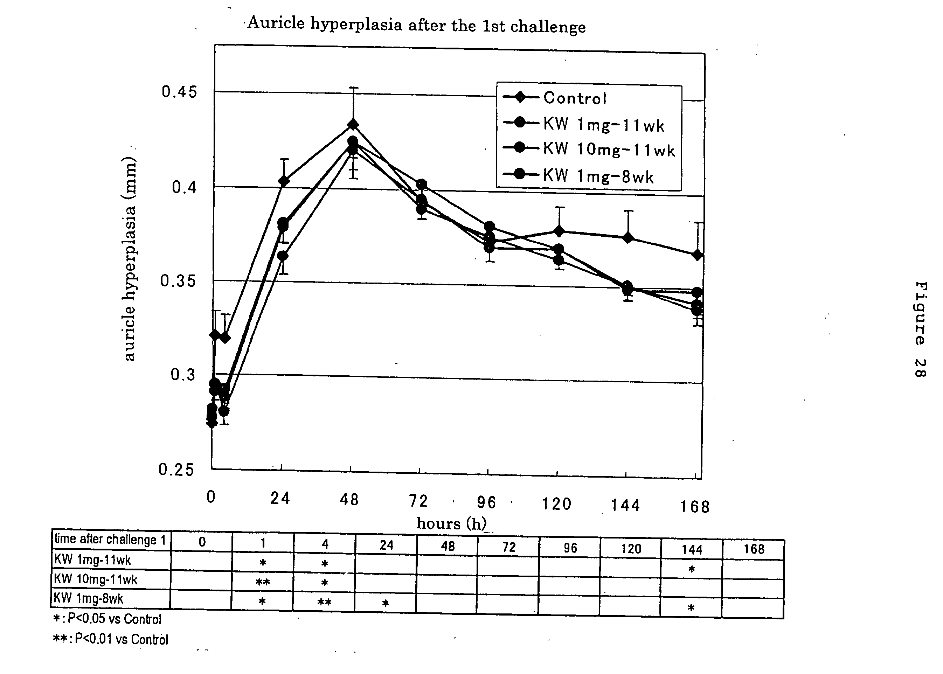

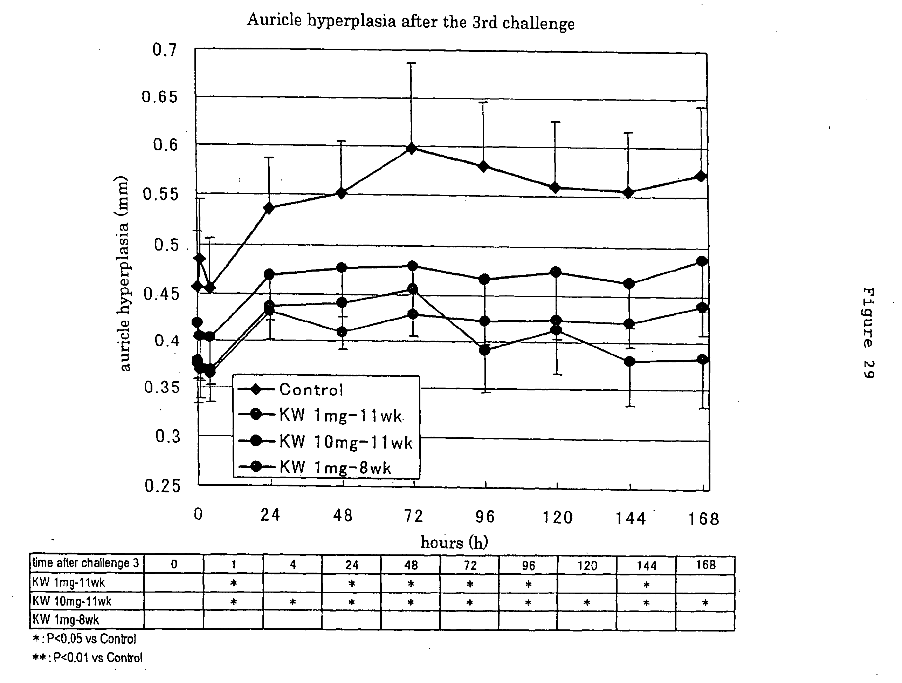

On the other hand, for all the three groups having ingested KW3110, significant suppression of thickening was observed at 1, 4 hours after the challenge that is said to be associated with immediate reaction. Further, a significant suppression of thickening was observed 144 hours after the challenge for KW10 mg-11 wk group, and 24 and 144 hours after the challenge for KW1 mg-8 wk group. After the third challenge, thickening was observed continuously after application for the control group, but for KW3110 ingesting group, a tendency of thickening suppression was observed as a whole, and a significant suppression was confirmed for KW1 mg-11 wk group and KW10 mg-11 wk group (Fig. 28, 29).

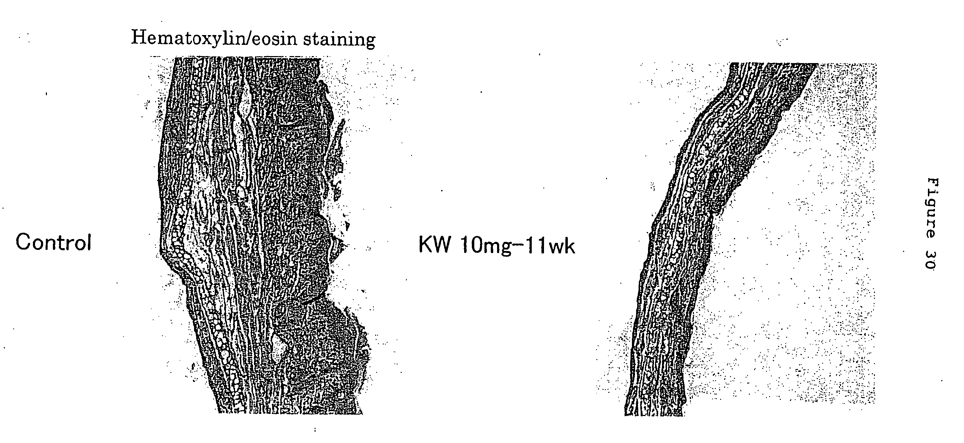

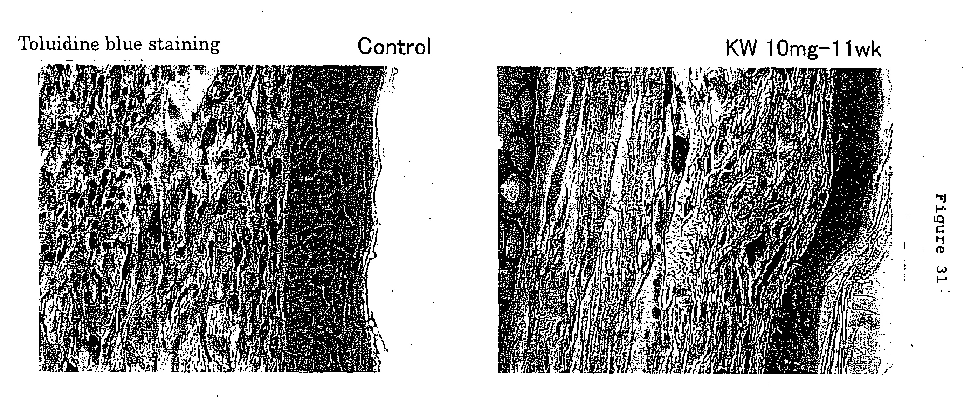

As a result of pathological estimation of the auricle part, for the control group, dermis and epidermis were thickened and inflammation such as edema was observed. However, for all the groups having ingested KW, it was observed that thickening was suppressed compared as a whole with the control group (Fig.30). Further, from pathological estimation by Toluidine blue staining, the number of mast cells of groups having ingested KW was confirmed to be less than that of the control group (Fig. 31).

From the above results, by ingesting KW, suppression of increase of total IgE concentration in blood, suppression of auricle thickening caused by hapten stimulation and suppression of deterioration of diseases at a clinical level were confirmed. Therefore, it was shown that by ingesting KW3110, the symptoms of hapten-induced atopic dermatitis were improved in NC/Ngamice.

### Example 10 (for reference only)

### (Effects of KW3110 strain in human for hay fever)

To investigate the effects of KW3110 strain in human, the following test was carried out to volunteers having hay fever.

### 1. Methods

### (Materials)

Prototype of yogurt prepared with *Lactobacillus delbrueckii* B strain that were estimated as E level for both Th1, Th2 parameters in Example 2 or *L. paracasei* KW3110 strain. Contains 2 × 10⁸ cfu/ml as for number of bacteria.

### (Target of the examination)

28 volunteers being hay fever patients working for an enterprise. The present examination was carried out after obtaining informed consent by writing, after explaining in detail to all test subjects, contents and methods of the test according to the declaration of Helsinki.

### (Test group)

28 test subjects were divided at random in two groups. One group ingested *L. delbrueckii* B strain yogurt, being the control yogurt, and the other group ingested *L. paracasei* KW3110 strain yogurt, 200 ml per day (corresponding to 40 mg of dried strain of KW3110 strain). As a result of classification, double-blind test was conducted wherein a third person controls the information until the end of test.

### (Test period)

From January 20, 2003 to April 14, 2003.

### (Measuring items)

Blood was collected from test subjects on week 0 (at the time of initiation of test) and at 4, 8, 12th week (at the termination of test). The following items of blood were measured at Falco Biosystem Ltd.: NK cell activity, ratio between the numbers of Th1 and Th2 cells (Th1/Th2 ratio), number of eosinophils, ECP level, total IgE level in blood and cedar pollen-specific IgE level in blood.

Data was organized after statistical processing for these data after the termination of test.

Moreover, test subjects answered a questionnaire as shown in the table at the time of collecting blood, in 5 levels (3+: high-level, 2+: moderate, +: mild; ±: slightly; -: none). 4 points for 3+, 3 points for 2+, 2 points for +, 1 point for ±, 0 point for -, and were calculated for statistical work.

**(Table 5)**

| item | symptom of chronic allergic rhinitis (hay fever) at the time of initiation | symptom of chronic allergic rhinitis (hay fever) after initiation |
|---|---|---|
| symptoms of running nose | 1.( 3+)2.( 2+ )3 . ( + ) 4. ( ± ) 5. ( - ) | 1 . ( 3 + ) 2 . ( 2+ )3. ( + ) 4. ( ± ) 5.( - ) |
| symptoms of stuffy nose | 1.( 3+ )2.( 2+ )3 . ( + ) 4.( ± ) 5. ( - ) | 1 . ( 3 + ) 2 . ( 2+ )3. ( + ) 4. ( ± ) 5. ( - ) |
| symptoms of sore throat | 1.( 3+ )2.( 2 + ) 3 . ( + ) 4.( ± ) 5.( - ) | 1.( 3+ )2 . ( 2 + ) 3 . ( + ) 4.( ± ) 5.( - ) |
| symptoms of itching of throat | 1.( 3+ )2.( 2+ )3. ( + ) 4.( ± ) 5.( - ) | 1.( 3+ )2.( 2+ )3. ( + ) 4.( ± )5.( - ) |
| symptoms of sneezing | 1.( 3+ )2.( 2+ )3. ( + ) 4.( ± )5.(-) | 1.( 3+ )2.( 2+ )3. ( + ) 4.( ± ) 5.( - ) |
| symptoms of eye irritation | 1.( 3+ )2.( 2+ )3. ( + ) 4.( ± ) 5. ( - ) | 1.( 3+ )2 . ( 2 + ) 3 . ( + ) 4.( ± ) 5. ( - ) |
| symptoms of hyperemia | 1.( 3+ )2.( 2+ )3 . ( + ) 4.( ± ) 5.( - ) | 1 . ( 3 + ) 2 . ( 2 + ) 3 . ( + ) 4.( ± ) 5 . ( - ) |
| symptoms of teary-eye | 1.( 3+ )2 . ( 2+ )3 . ( + ) 4.( ± ) 5 . ( - ) | 1 . ( 3 + ) 2 . ( 2 + ) 3 . ( + ) 4.( ± ) 5 . ( - ) |
| symptoms of eye pain | 1.( 3+ )2 . ( 2 + ) 3 . ( + ) 4.( ± ) 5 . ( - ) | 1 . ( 3 + ) 2 . ( 2 + ) 3 . ( + ) 4.( ± ) 5 . ( - ) |
| symptoms of eye mucus | 1.( 3+ ) 2.( 2 + ) 3. ( + ) 4.( ± ) 5. ( - ) | 1 . ( 3 + ) 2 . ( 2 + ) 3 . ( + ) 4.( ± ) 5 . ( - ) |
| symptoms of heavy feelings in eyelids | 1.( 3+ )2.( 2+ )3. ( + ) 4.( ± ) 5. ( - ) | 1.( 3+ )2.(2+)3. ( + ) 4.( ± ) 5.( - ) |
| symptoms of heavy feeling of head | 1.( 3+ )2.(2+)3. ( + ) 4.( ± ) 5. ( - ) | 1.( 3+ )2.( 2+ )3. ( + ) 4.( ± ) 5.( - ) |
| symptoms of feeling tired | 1.( 3+ )2.( 2+ )3. (+) 4.(±) 5.(-) | 1.( 3+ )2.( 2+ )3. (+) 4.(±) 5.(-) |

**(Table 6)**

| item | subjective syptoms |
|---|---|
| 3+: high-level symptoms | High-level symptoms are observed. For example, frequency and quantity of running nose is high, and hard to work or study. |
| 2+: moderate symptoms | Moderate symptoms are observed. For example, frequency increases and it is bothering. |
| +: mild symptoms | Mild symptoms are observed. For example, frequency is low and it is not much bothering. |
| ±: slight symptoms | Slight symptoms are observed. For example, creepy sometimes but it is resistible. |
| -: no symptoms | Almost none or no symptoms. Not bothering |

### 2. Results

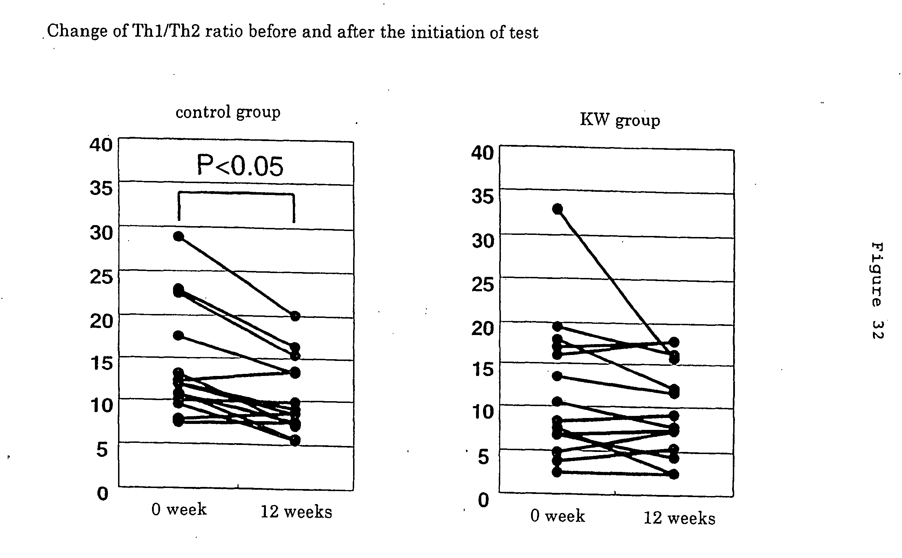

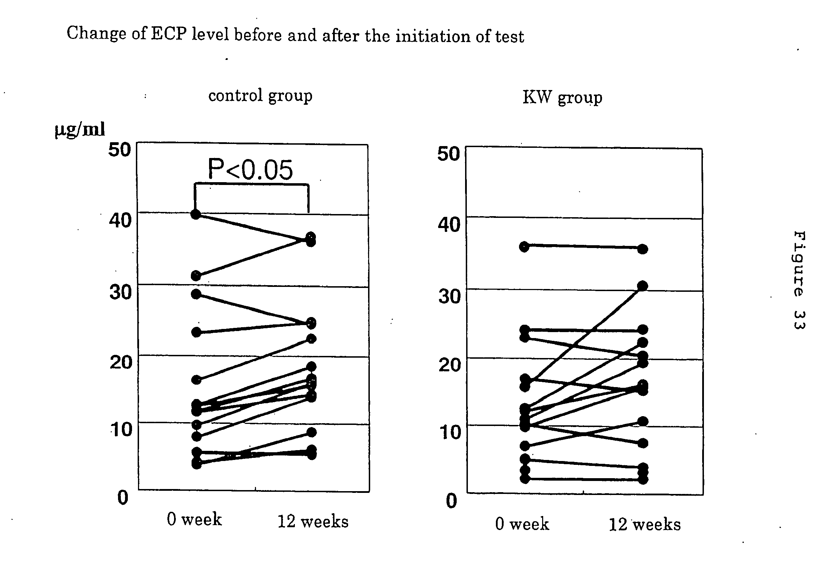

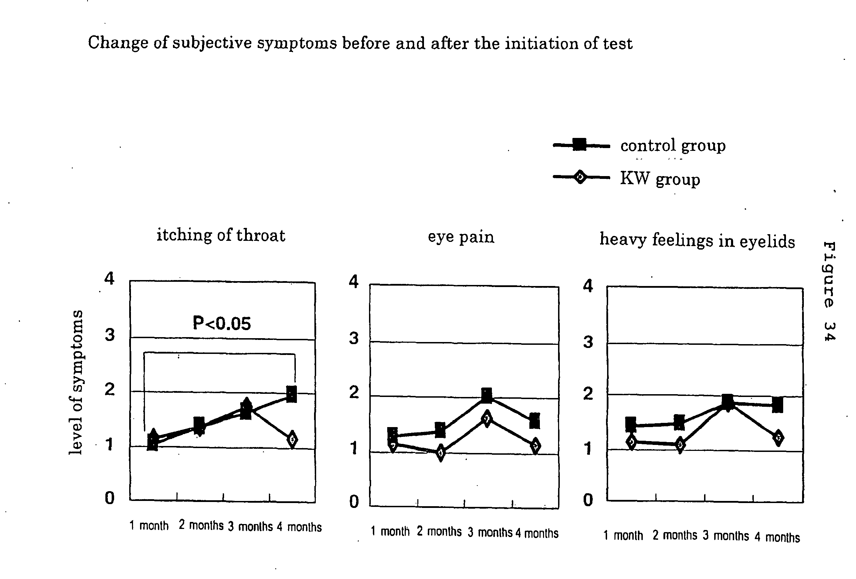

By comparing the results at the initiation of the test with that of at the termination, for *L. delbruckii* B strain yogurt group (control group) which is the control, as it is shown in Fig. 32, Th1/Th2 ratio showed a significant decrease and as it is shown in Fig. 33, ECP level showed a significant increase. On the other hand, for *L. paracasei* KW3110 strain yogurt group (KW group), there were no significant change between before and after the test for both levels. Moreover, as it is shown in Fig. 34, as for the score of subjective symptoms, for the items such as itching of throat, eye pain and heavy feeling in eyelids, KW group showed lower levels compared with control group. From the above results, effectiveness of KW3110 strain was also shown for human hay fever.

In the following, examples wherein the lactic acid bacteria with antiallergic activity are applied for production of beverages are described.

### Example 11 (for reference only)

### (Methods for preparing the lactic acid bacteria strain as material to be added to beverages)

### Example of preparation 1. Preparation of the lactic acid bacteria KW3110 strain

Culture medium (glucose 1%, yeast extract S (Takeda-Kirin Foods Corporation) 1%, MgSO₄· 7H₂O 50 ppm, MnSO₄·5H₂O 50 ppm) was used to culture the lactic acid bacteria. Preculture was conducted by inoculating the lactic acid bacteria KW3110 strain in 10 ml of culture medium and by standing at 37°C for 20-24 hours. For the culture, 0.6 ml of preculture solution was inoculated to 120 ml of culture medium, and by using a 200 ml-fermenter (BMJ-25 model, Able) it was cultured at 28°C. Aeration level was 0.12 L/min, stirring speed was 500 rpm. By using 25% NaOH solution pH was controlled not to be 4.5 or less. It was cultured for 48 hours.

After the termination of the culture, by centrifuging for 10 minutes at 8500Xg, strains were harvested. Strains were suspended to 30 ml sterile distilled water, and by centrifugation at 8500Xg for 10 minutes, strains were harvested. The washing process was repeated for three times, and the compositions derived from medium were removed. The washed strains were suspended to 10 ml sterile water, treated in an autoclave at 100°C for 30 minutes, then lyophilized. Finally, 50-70 mg of the lactic acid bacteria dried strain were obtained. The antiallergic activity of the obtained lactic acid bacteria strain was confirmed.

### Example 12 (for reference only)

### (Preparation of tea drinks)

Deionized water was added to extract of oolong tea, red tea, green tea, roasted green tea and jasmine tea extracted with hot water of 85°C, so that the usage rate of tea leaf becomes 0.8 weight %. At that time, ascorbic acid was added so that it becomes 0.025 weight %, and pH was controlled by using sodium bicarbonate to be easy to drink. Further, the lactic acid bacteria KW3110 strain were added so that the number of bacteria becomes 1.5 × 10¹⁰, 3 x 10¹⁰ per 100 g of the preparation, UHT sterilization was carried out by a common procedure, and filled in a 350 ml PET bottle.

These ten examples (5 types of tea × 2 stages of lactic acid bacteria concentration) were subjected to a sensory examination by panelists. As a result, all the beverages had satisfactory flavor and received high evaluation that it can be drunk everyday continuously. However, for samples wherein 3 × 10¹⁰ was added, as the lactic acid bacteria are noticeable as precipitate, it was estimated that samples wherein 1.5 x 10¹⁰ was added are preferable.

### Example 13 (for reference only)

### (Preparation of barley tea)

250 g of Chinese pearl barley tea was decocted with 10 kg of boiling water for 30 min, filtered and centrifuged. The lactic acid bacteria KW3110 strain was added to the obtained extract so that it would be 8 × 10¹⁰, 1.5 x 10¹⁰, 3 x 10¹⁰ per 100 g of the final product, and further, sodium bicarbonate and ascorbic acid were added to adj ust the taste, and deionized water was added to obtain 10 kg in weight once more. Then, UHT sterilization was conducted by a common procedure, and it was filled in a 500 ml PET bottle aseptically.

By substituting to sensory evaluation, all of the beverages had satisfactory flavor and received high evaluation that it can be drunk everyday continuously. However, for samples wherein 3 x 10¹⁰ was added, as the lactic acid bacteria are noticeable as precipitate in appearance, it was estimated that samples wherein 1.5 x 10¹⁰ was added are more preferable.

### Example 14 (for reference only)

### (Preparation of beverage containing juice)

A prescribed amount of the lactic acid bacteria strain was added to 900 g of cloudy apple juice and 1g of flavor, and further deionized water was added so that the total become 1 kg. By a common procedure, it was hot pack filled in a 180 ml glass bottle to prepare beverage containing apple juice. Meanwhile, the lactic acid bacteria KW3110 strain were added so that the number of bacteria would be 2 × 10¹⁰, or 4 x 10¹⁰ per 100 g of the preparation.

As a result of sensory evaluation, there were absolutely no unpleasant sensation of flavor due to the lactic acid bacteria compounding, neither no problem in appearance for all the samples.

### Example 15 (for reference only)

### (Preparation of sport drink)

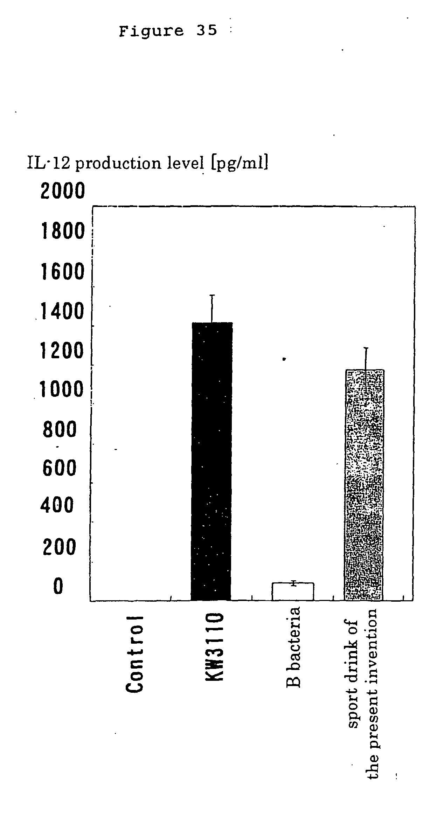

0.0143 weight % of the lactic acid bacteria KW3110 strain (1.5 x 10¹⁰ per 100 g of product) was added to preparation comprising 4.5 weight % of granulated sugar, 0.2 weight % of citric acid, 0.05 weight % of sodium citrate and 0.1 weight % of flavor were added. Further, the solution was heated up to 90°C for sterilization, hot packed into PET bottle to prepare a beverage of sport drink type. As a result of sensory evaluation, both appearance and flavor were satisfactory. The lactic acid bacteria strain in the beverage were centrifuged, washed twice with purified water, then lyophilized to obtain dried strain. The finished products were estimated in the *in vitro* systems by using mouse lymphocytes, and it was confirmed the prescribed activity was maintained (Fig. 35).

### Example 16 (for reference only)

### (Preparation of tomato juice)

After adding a prescribed amount of the lactic acid bacteria strain to tomato juice, the preparation was filled into a 190 g can, and according to a common procedure, retort sterilization was performed. Meanwhile, the lactic acid bacteria KW3110 strain was added so that the number of strain would be 2.5 x 10¹⁰ or 5 × 10¹⁰ per 100 g of the preparation.

As a result of sensory evaluation, there were absolutely no unpleasant sensation of flavor due to the lactic acid bacteria compounding, neither no problem in appearance for all the samples.

### Example 17

### (Preparation of coffee) (for reference only)

Roasted and ground coffee beans (Columbia) were extracted with hot water of 95°C and coffee extract was obtained. By adding deionized water, the extract was diluted so that the soluble solid content becomes 1.4%. The lactic acid bacteria KW3110 strain were added so that the number of bacteria would be 2 x 10¹⁰ or 4 x 10¹⁰ per 100 g of the preparation, filled in a 190 g can and according to a common procedure, retort sterilization was carried out.

As a result of sensory evaluation, there were absolutely no unpleasant sensation due to the lactic acid bacteria compounding for all of the samples. However, when pouring into glasses and the like, the lactic acid bacteria were noticeable in appearance as precipitant for samples wherein 4 × 10¹⁰ was added, and it was estimated that preparation wherein 1.5 x 10¹⁰ was added are more preferable. However, there was no problem when drunk directly from the can.

In the following, examples wherein the lactic acid bacteria with antiallergic activity of the present invention are applied for production of foods are described.

### Example 18

### (Preparation of yogurt)

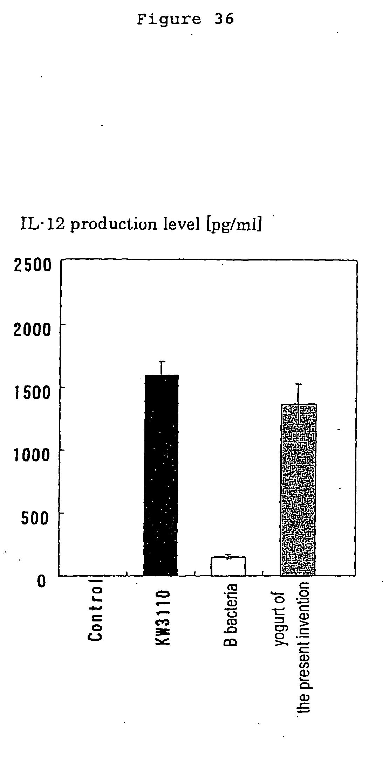

Raw materials containing milk (milk, skim milk, cream, sugar liquid, stabilizer, flavor, water) were mixed homogeneously, sterilized by heating at 128°C for 15 seconds, cooled down to 40°C or less, added with the lactic acid bacteria starter KW3110, and started fermentation in a fermentation tank maintained at 37°C. The lactic acid bacteria KW3110 decomposed lactose in the mixture to generate lactic acid, and in about 18 hours, pH became 4.6, and by isoelectric aggultination of milk protein, stirred and cooled when the gelation stabilized. Yogurt made in form of paste by stirring, was filled in a paper container, sealed and stored being chilled in a refrigerator of 10°C or less. After cooling, by a sensory evaluation, the mixture was confirmed to have appropriate eating quality and properties as yogurt. The compounding proportion of raw materials for yogurt is shown in table 7. Moreover, the proportion of fat and SNF (solids-not-fat) in raw materials and the sweetness are shown in Table 8. The finished products were estimated in the *in vitro* systems by using mouse lymphocytes, and it was confirmed that the prescribed activity was maintained (Fig. 36).

**(Table 7)**

| raw materials | (weight %) |
|---|---|
| milk | 50.00 |
| skim milk | 4.63 |
| cream | 2.42 |
| sugar liquid | 9.50 |
| stabilizer | 0.80 |
| flavor | 0.08 |
| lactic acid bacteria starter | 5.00 |
| water | 27.57 |
| total | 100.00 |

**(Table 8)**

| proportion of particular composition in raw materials (%) and sweetness | |
|---|---|
| fat | 3.1% |
| SNF | 9.3% |
| sweetness | 6.4 |

In the following, examples wherein the lactic acid bacteria with antiallergic activity are applied for production of health food products are described.

### Example 19 (for reference only)

### (Preparation of health foods in tablet)

Reduced palatinose,sorbitol,xylitoland gum alabic being sugars, were added appropriately to 67 g of prepared dried powder of the lactic acid bacteria KW3110 strain, by heating and granulating, by using water as binder. Taste was adjusted with citric acid, stevia, flavor and the like, powdered fat and sucrose ester were added so that it is easy to form into tablets by using a compressing machine, and obtained a mixture of 1000 g. Then, the mixture was compressed to be 1.5 g per tablet, and health foods in form of tablet favorable in flavor were prepared. At that time, the lactic acid bacteria KW3110 strain were 5 × 10¹⁰ per tablet.

### Example 20 (for reference only)

### (Health food in form of hard capsule)

50 g of dextrin was added to 100 g of prepared dried powder of the lactic acid bacteria KW3110 strain, and after being mixed homogeneously, the mixture was filled into base materials of hard capsule comprising pullulan, vegetable oil, carrageenan, potassium chloride to obtain heath foods of 213 mg per capsule encapsulated with hard capsules. At that time, the lactic acid bacteria KW3110 strain were contained by 5 x 10¹⁰ per capsule.

### Example 21 (for reference only)

### (Health food in form of hard capsule)

50 g of dextrin was added to 100 g of prepared dried powder of the lactic acid bacteria KW3110 strain, and after being mixed homogeneously, the mixture was filled into base materials of hard capsule comprising gelatin and glycerin, to obtain heath foods of 213 mg per capsule encapsulated with hard capsules. At that time, the lactic acid bacteria KW3110 strain were 5 x 10¹⁰ per capsule.

### Example 22 (for reference only)

### (Health food in form of soft capsule)

100 kg of prepared dried powder of the lactic acid bacteria KW3110 strain were suspended homogeneously with a mixture of 180 kg of safflower oil, 20 kg of bees wax and 5 kg of soybean lecithin, encapsulated with base material of capsules having as main ingredients carrageenan, starch and glycerine to form a soft capsule having an oval form of 450 mg per capsule. At that time, the lactic acid bacteria KW3110 strain were 5 × 10¹⁰ per capsule.

### Example 23 (for reference only)

### (Health food in form of soft capsule)

100 kg of prepared dried powder of the lactic acid bacteria KW3110 strain were suspended homogeneously with a mixture of 180 kg of safflower oil, 20 kg of bees wax and 5 kg of soybean lecithin, encapsulated with base material of capsules comprising gelatin and glycerin to form a soft capsule having an oval form of 450 mg per capsule. At that time, the lactic acid bacteria KW3110 strain were 5 x 10¹⁰ per capsule.

### Example 24 (for reference only)

### (Health food in form of granule)

100 kg of prepared dried powder of the lactic acid bacteria KW3110 strain were filled with a stick filling apparatus to be 1 g per stick, to obtain health food wherein the lactic acid bacteria KW3110 strain are 5 x 10¹¹ per stick.

### Example 25 (for reference only)

### (Health food in form of granule)

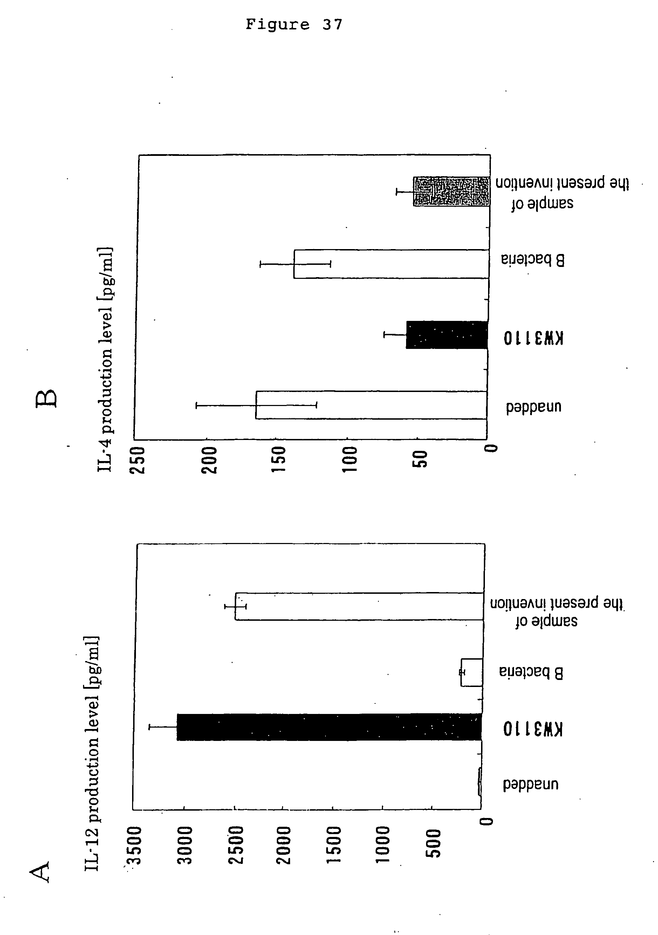

900 kg of dextrin were added to 100 kg of prepared dried powder of the lactic acid bacteria KW3110 strain, and by using water as binder, with a fluid bed granulator, mixing, heating and granulating were carried out homogeneously to obtain 1000 kg of granules. Then the granules were filled with a stick filling apparatus to be 1 g per stick, to obtain health foods wherein the lactic acid bacteria KW3110 strain are 5 × 10¹⁰ per stick. The finished products were estimated in the *in vitro* systems by using mouse lymphocytes, and it was confirmed that the prescribed activity was maintained (Fig. 37).

### Example 26 (for reference only)

### (Powdered health food)

900 kg of dextrin were added to 100 kg of prepared dried powder of the lactic acid bacteria KW3110 strain, and by using water as binder, with a fluid bed granulator, mixing, heating, and granulating were carried out homogeneously to obtain 1000 kg of granules. Then the granules were ground until the total amount passed No. 18 pole, filled with a stick filling apparatus to be 1 g per stick, to obtain powdered health foods wherein the lactic acid bacteria KW3110 strain are 5 x 10¹⁰ per stick.

### Example 27 (for reference only)

### (Health food in form of suspended solution)

100 g of prepared dried powder of the lactic acid bacteria KW3110 strain were added to 10 kg of pressed juice of kale leaf, adjusted the viscosity with 10 g sodium alginate so that the lactic acid bacteria can be dispersed easily to obtain health food in form of suspended solution.

### Example 28 (for reference only)

### (Health food in form of suspended solution)

100 g of prepared dried powder of the lactic acid bacteria KW3110 strain were mixed with 0.7 kg of glucose, 5 kg of distilled water and 5 g of grape flavor, and after sterilization by heating, the mixture was filled aseptically in a 50 ml sealed container to obtain health food in form of suspended solution as a syrup. This preparation had good reputation to children by not giving unpleasant sensation.

### Brief Description of the Drawings

Fig. 1 is a list showing the lactic acid bacteria used in the examples and the place obtained (No.1).
Fig. 2 is a list showing the lactic acid bacteria used in the examples and the place obtained (No.2).
Fig. 3 is a graph showing the production level of IL-12, which is Th1 cytokine, by various lactic acid bacteria in the examples.
Fig. 4 is a graph showing the production level of IL-4, which is Th2 cytokine, by various lactic acid bacteria in the examples.
Fig. 5 is a set of graphs showing the results of comparison of the IL-12 and IL-4 production levels *in vitro,* by using the lactic acid bacteria strain of the present invention (KW3110) and the comparative strain (L-92 strain) in the examples.
Fig. 6 is a graph showing the results of measuring the antiallergic activity of strain No. 90 derived from KW3110 strain, selected in the examples.
Fig. 7 is a figure showing the experiment schedule of the examination for measuring antiallergic ability *in vivo* of KW3110 strain, being the lactic acid bacteria strain showing high antiallergic activity of the present invention, in the examples.

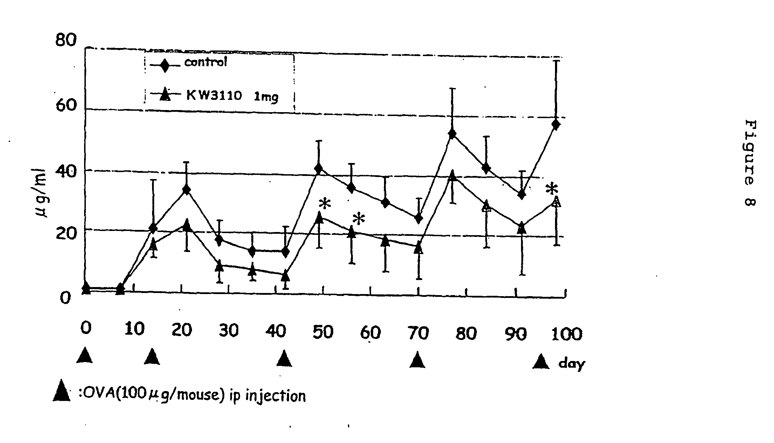

Fig. 8 is a graph showing the variation of IgE in blood in an experiment of 98 days, to measure antiallergic ability *in vivo* of KW3110 strain, being the lactic acid bacteria strain showing high antiallergic activity of the present invention, in the examples.

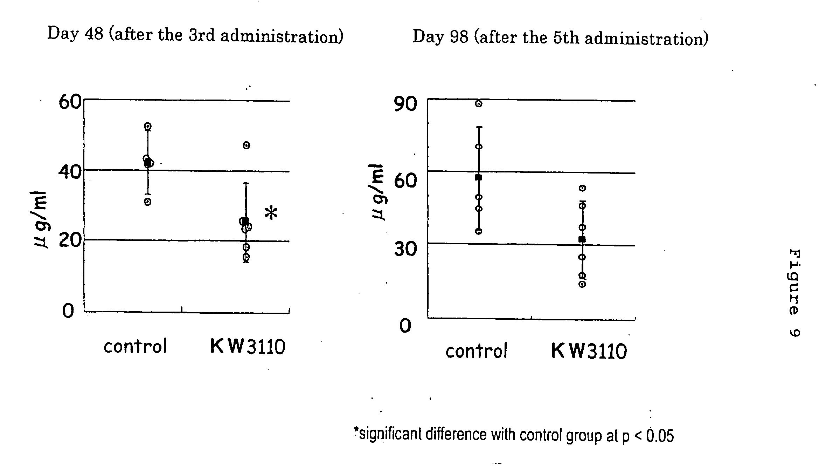

Fig. 9 is a set of graphs showing IgE level in blood by dot-blot on day 48 and on day 98 at the time of dissection, in an experiment to measure antiallergic activity *in vivo* of KW3110 strain, being the lactic acid bacteria strain showing high antiallergic activity of the present invention, in the examples.

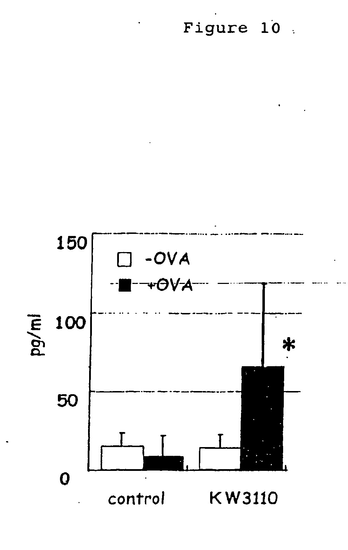

Fig. 10 is a graph showing IL-12 production levels of the control group and KW3110 group in the splenic cell culture on day 98 at the time of dissection, in an experiment to measure antiallergic activity *in vivo* of KW3110 strain, being the lactic acid bacteria strain showing high antiallergic activity of the present invention, in the examples.

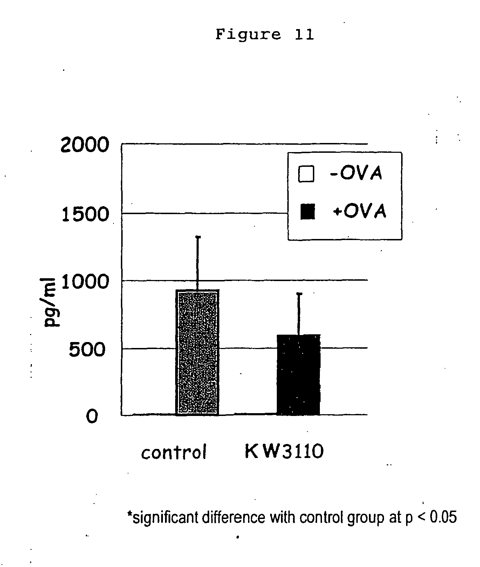

Fig. 11 is a graph showing IL-4 production levels of the control group and KW3110 group in the splenic cell culture on day 98 at the time of dissection, in an experiment to measure antiallergic activity *in vivo* of KW3110 strain, being the lactic acid bacteria strain showing high antiallergic activity of the present invention, in the examples.

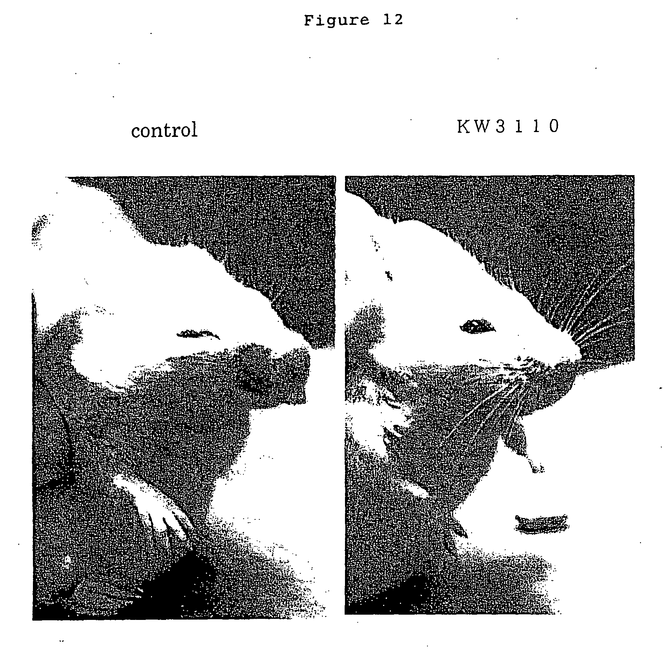

Fig. 12 is a set of pictures showing the results of observing hair loss around the nose of the control group and KW3110 group, in an experiment to measure antiallergic activity *in vivo* of KW3110 strain, being the lactic acid bacteria strain showing high antiallergic activity of the present invention, in the examples.
Figure 13 is a figure showing the experiment schedule of the experiment for estimating preventive effect using feed containing KW3110 strain being the lactic acid bacteria showing high antiallergic activity of the present invention and feed containing KW4610 strain being publicly known as antiallergic lactic acid bacteria, for comparison between KW3110 strain and KW4610 strain, in the examples.
Fig. 14 is a graph showing the variation of IgE level in blood during the experiment for estimating preventive effect using feed containing KW3110 strain being the lactic acid bacteria showing high antiallergic activity of the present invention and feed containing KW4610 strain being publicly known as antiallergic lactic acid bacteria, for comparison between KW3110 strain and KW4610 strain, in the examples.
Fig. 15 is a set of graphs showing IgE level in blood by dot per individual after 5th and 6th administration of OVA, in the experiment for estimating preventive effect using feed containing KW3110 strain being the lactic acid bacteria showing high antiallergic activity of the present invention and feed containing KW4610 strain being publicly known as antiallergic lactic acid bacteria, for comparison between KW3110 strain and KW4610 strain, in the examples.
Fig. 16 is a graph showing the results of the acid-resistance test of KW3110 strain being the lactic acid bacteria showing high antiallergic activity of the present invention, in the examples of the present invention.
Fig. 17 is a graph showing the results of the bile acid-resistance test of KW3110 strain being the lactic acid bacteria showing high antiallergic activity of the present invention, in the examples of the present invention.
Fig. 18, Fig. 18 a, b, c are graphs showing the results of examining sneezing behavior of the mouse models, for the improvement effects of KW3110 strain on hay fever/airway inflammation, by using pollen antigen, in the examples.
Fig. 19, Fig. 17 a, b are graphs showing the results of examining scratching behavior of the mouse models, for the improvement effects of KW3110 strain on hay fever/airway inflammation, by using pollen antigen, in the examples.
Fig. 20 is a graph showing the results of examining the suppression of increase of total IgE concentration in blood of mouse models, for the improvement effects of KW3110 strain on hay fever/airway inflammation, by using pollen antigen, in the examples.
Fig. 21 is a set of graphs showing the results of examining the suppression of increase rate of eosinophil (Fig. 19 a) and the suppression of topical invasion of eosinophil (Fig. 19 b) of mouse model, for the improvement effects of KW3110 strain on hay fever/airway inflammation, by using pollen antigen, in the examples.
Fig. 22 is a graph showing the results of examining IL-5 level in mouse models, for the improvement effects of KW3110 strain on hay fever/airway inflammation, byusingpollen antigen, in the examples.
Fig. 23 is a set of graph and table showing the results of examining the increase of the total IgE concentration, for the improvement effects of KW3110 strain on mouse models showing symptoms of atopic dermatitis, in the examples.
Fig. 24 is a set of graph and table showing the measurement results of total clinical scores of mouse models, for the improvement effects of KW3110 strain on mouse models showing symptoms of atopic dermatitis, in the examples.

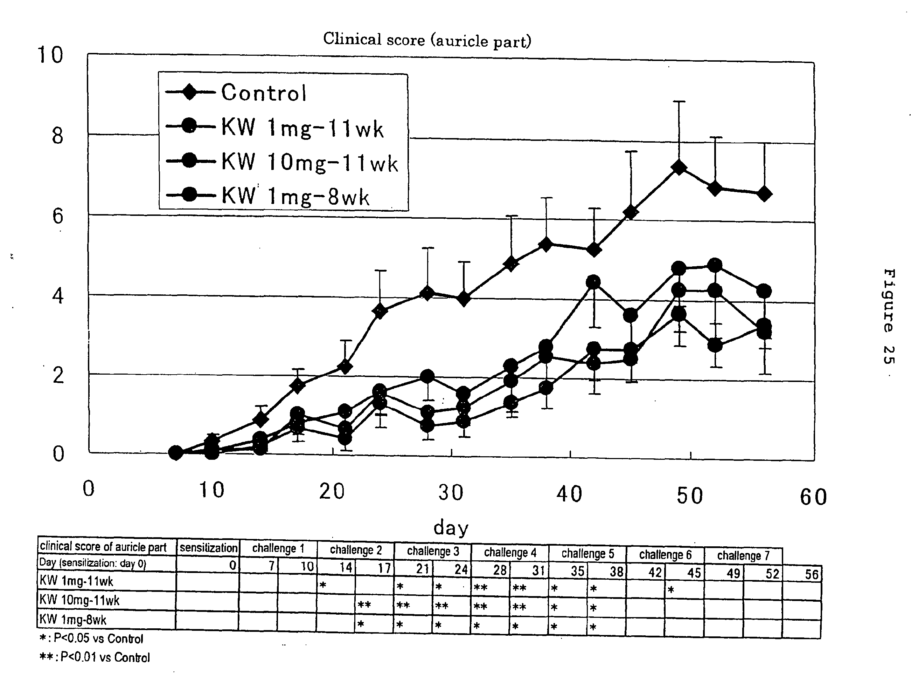

Fig. 25 is a set of graph and table showing the measurement results of clinical scores of mouse models (auricle part), for the improvement effects of KW3110 strain on mouse models having atopic dermatitis, in the examples.

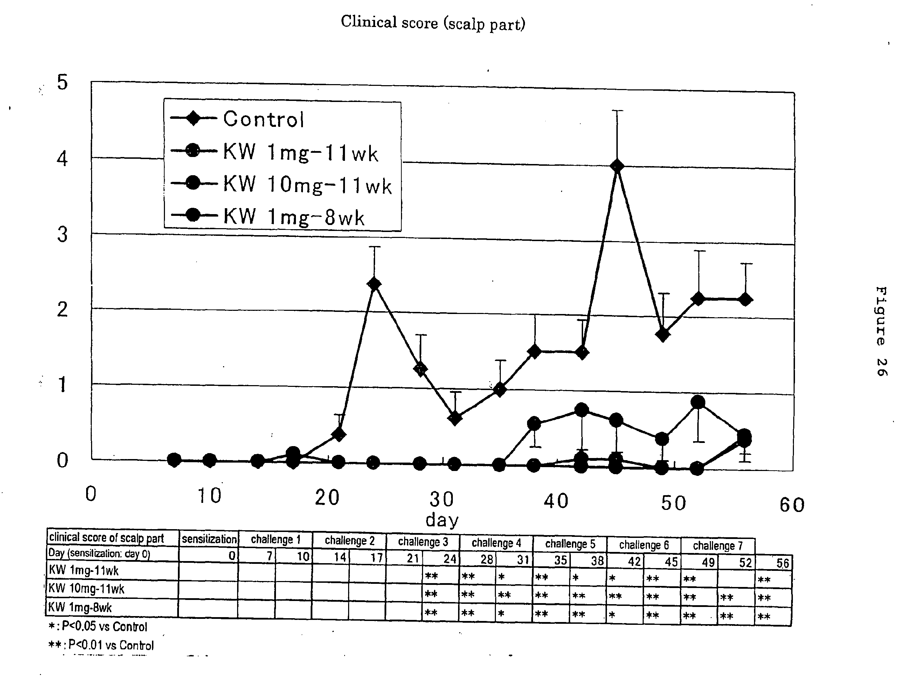

Fig. 26 is a set of graph and table showing the measurement results of clinical scores of mouse models (scalp part), for the improvement effects of KW3110 strain on mouse models having atopic dermatitis, in the examples.
Fig. 27 is a set of pictures showing the suppression state of erosion/damage of tissues of auricle part, in the head of mouse models, for the improvement effects of KW3110 strain on mouse models having atopic dermatitis, in the examples.
Fig. 28 is a set of graph and table showing the experimental results of the auricle thickening of mouse model (Fig. 26a) and suppressing trend of auricle thickening of mouse model after the first challenge (Fig. 26b), for the improvement effects of KW3110 strain on mouse models having atopic dermatitis, in the examples.
Fig. 29 is a set of graph and table showing the results of examining the suppressing trend of auricle thickening of mouse models after the third challenge, for the improvement effects of KW3110 strain on mouse models having atopic dermatitis, in the examples.
Fig. 30 is a set of pictures showing the results of pathological estimation of auricle part by using hematoxylin eosin staining for the suppression of auricle thickening of mouse models, for the improvement effects of KW3110 strain on mouse models having atopic dermatitis, in the examples.
Fig. 31 is a set of pictures showing the pathological estimation of auricle part by using Toluidine blue staining for the suppression of auricle thickening of mouse models, for the improvement effects of KW3110 strain on mouse models having atopic dermatitis, in the examples.
Fig. 32 is a set of graphs showing the results of measuring the change of Th1/Th2 ratio, in the test using yogurt, for the effects of KW3110 strain on hay fever in human, in the examples.
Fig. 33 is a set of graphs showing the results measuring the change of ECP before and after the initiation of the test, in the test using yogurt, for the effects of KW3110 strain on hay fever in human, in the examples.
Fig. 34 is a set of graphs showing the results of observing the change of subjective symptoms before and after the initiation of the test, in the test using yogurt, for the effects of KW3110 strain for hay fever in human, in the examples .
Fig.35 is a graph showing the results of examining IL-12 production level when manufactured as a soft drink, examining the maintenance of antiallergic activity when manufactured by conducting various treatments, by using KW3110 strain, in the examples.
Fig. 36 is a graph showing the results of examining IL-12 production level when manufactured by compounding in yogurt, examining the maintenance of antiallergic activity when manufactured by conducting various treatments, by using KW3110 strain, in the examples.
Fig. 37 is a graph showing the result of examining IL-12 and Il-4 production levels when manufactured as tablets, examining the maintenance of antiallergic activity when manufactured by conducting various treatments, by using KW3110 strain, in the examples.

### Industrial Applicability

It is possible to obtain the lactic acid bacteria with high antiallergic activity, and to have compositions showing high antiallergic activity having the lactic acid bacteria as active ingredients. The reference antiallergic composition is particularly effective to environmental allergy such as hay fever, atopic dermatitis, bronchial asthma, allergic rhinitis, allergic conjunctivitis and the like. Particularly, as it is shown in the data of hay fever or data of therapy experiments for asthma using pollen as an antigen, it shows significant effects on prevention/treatment of diseases of mucosal hypersensivity, such as hay fever, bronchial asthma, allergic rhinitis, allergic conjunctivitis and the like. *L. paracasei* KW3110, being one of the active ingredients of the antiallergic composition of the present invention, not only has strong antiallergic activity, immunostimulating activity but also are excellent for its adhesiveness to intestinal cells and resistance to gastric acid and bile acid. Therefore, by administering orally the lactic acid bacteria or products using the lactic acid bacteria *L. paracasei* KW 3110, the use of foods or drinks that can exercise functions of probiotics that are conventionally known, such as function regulating intestines, function lowering cholesterol, or hypotensive function shown in many lactic acid bacteria, besides antiallergic function. The composition with antiallergic function can be ingested every day continuously, particularlyinformofbeverage, and alsomaintain antiallergic function that become effective with the amount possible to ingest continuously. Moreover, it is possible to provide beverages of good taste and good storage ability.

## Claims

1. A probiotics product comprising a viable lactic acid bacteria Lactobacillus paracasei KW3110 strain or its mutant as an active ingredient, wherein the probiotics product is a beverage or food product.

2. The probiotics product according to claim 1, wherein the probiotics product is a dairy raw material fermented by using the lactic acid bacteria Lactobacillus paracasei KW3110 strain or its mutant.

3. The probiotics product according to claim 1 to 2, wherein the probiotics product is yogurt.

4. The probiotics product according to claim 1 to 3, wherein the number of the lactic acid bacteria Lactobacillus paracasei KW3110 strain or its mutant in the probiotics product is 5 X 10⁹ or more cells per daily intake.

5. The probiotics product according to claim 1 to 4, wherein the probiotics product is used for preventing and/or treating allergic diseases.

6. A method for producing probiotics products, wherein a part or whole of the lactic acid bacteria in the beverage or food produced by using lactic acid bacteria or the beverage or food comprising lactic acid bacteria is replaced by the lactic acid bacteria Lactobacillus paracasei KW3110 strain or its mutant.

7. A method for producing a probiotics product comprising the lactic acid bacteria, wherein the lactic acid bacteria is Lactobacillus paracasei KW3110 strain or its mutant.

8. A method for producing a probiotics product which is produced by using lactic acid bacteria, wherein the used lactic acid bacteria is Lactobacillus paracasei KW3110 strain or its mutant.

9. The method for producing a probiotics product according to claims 6 to 8, wherein the probiotics product is a beverage or food product.

10. The method for producing a probiotics product according to claim 9, wherein the probiotics product is a dairy raw material fermented by using the lactic acid bacteria Lactobacillus paracasei KW3110 strain or its mutant.

11. The method for producing a probiotics product according to claims 9 to 10, wherein the probiotics product is yogurt.

12. The method for producing a probiotics product according to claims 6 to 11, wherein the number of the lactic acid bacteria Lactobacillus paracasei KW3110 strain or its mutant in the probiotics product is 5 X 10⁹ or more cells per daily intake.

13. The method for producing a probiotics product according to claims 6 to 12, wherein the probiotics product is used for preventing and/or treating allergic diseases.

14. Use of lactic acid bacteria Lactobacillus paracasei KW3110 strain or its mutant in the manufacture of a probiotics product.

15. The use of lactic acid bacteria Lactobacillus paracasei KW3110 strain or its mutant in the manufacture of a probiotics product according to claim 14, wherein the probiotics product is a beverage or food product.

16. The use of lactic acid bacteria Lactobacillus paracasei KW3110 strain or its mutant in the manufacture of a probiotics product according to claim 15, wherein the probiotics product is a dairy raw material fermented by using the lactic acid bacteria Lactobacillus paracasei KW3110 strain or its mutant.

17. The use of lactic acid bacteria Lactobacillus paracasei KW3110 strain or its mutant in the manufacture of a probiotics product according to claims 15 and 16, wherein the probiotics product is yogurt.

18. The use of lactic acid bacteria Lactobacillus paracasei KW3110 strain or its mutant in the manufacture of a probiotics product according to claims 14 to 17, wherein the number of the lactic acid bacteria Lactobacillus paracasei KW3110 strain or its mutant in the probiotics product is 5 X 10⁹ or more cells per daily intake.

19. The use of lactic acid bacteria Lactobacillus paracasei KW3110 strain or its mutant in the manufacture of a probiotics product according to claims 14 to 18, wherein the probiotics product is used for preventing and/or treating allergic diseases.

## Patentansprüche

1. Probiotisches Produkt, umfassend einen wachstumsfähigen KW3110-Stamm des Milchsäurebakteriums Lactobacillus paracasei oder seine Mutante als einen wirksamen Bestandteil, worin das probiotische Produkt ein Getränk oder Nahrungsmittel ist.

2. Probiotisches Produkt gemäß Anspruch 1, worin das probiotische Produkt ein Rohmaterial einer Molkerei ist, das unter Verwendung des KW3110-Stamms des Milchsäurebakteriums Lactobacillus paracasei oder seiner Mutante fermentiert ist.

3. Probiotisches Produkt gemäß den Ansprüchen 1 bis 2, worin das probiotische Produkt ein Joghurt ist.

4. Probiotisches Produkt gemäß Anspruch 1 bis 3, worin die Anzahl des KW3110-Stamms des Milchsäurebakteriums Lactobacillus paracasei oder seiner Mutante im probiotischen Produkt 5 x 10⁹ oder mehr Zellen pro Tagesaufnahme ist.

5. Probiotisches Produkt gemäß Anspruch 1 bis 4, worin das probiotische Produkt zum Vorbeugen und/oder Behandeln von allergischen Erkrankungen verwendet wird.

6. Verfahren zum Herstellen probiotischer Produkte, worin ein Teil oder die Gesamtheit der Milchsäurebakterien in dem Getränk oder dem Nahrungsmittel, das unter Verwendung von Milchsäurebakterien hergestellt wurde, oder dem Getränk oder dem Nahrungsmittel, das Milchsäurebakterien umfaßt, durch den KW3110-Stamm des Milchsäurebakteriums Lactobacillus paracasei oder seine Mutante ersetzt wird.

7. Verfahren zum Herstellen eines probiotischen Produkts, umfassend das Milchsäurebakterium, worin das Milchsäurebakterium der Lactobacillus paracasei KW3110-Stamm oder seine Mutante ist.

8. Verfahren zum Herstellen eines probiotischen Produkts, das unter Verwendung eines Milchsäurebakteriums hergestellt wird, worin das verwendete Milchsäurebakterium der Lactobacillus paracasei KW3110-Stamm oder seine Mutante ist.

9. Verfahren zum Herstellen eines probiotischen Produkts gemäß den Ansprüchen 6 bis 8, worin das probiotische Produkt ein Getränk oder Nahrungsmittel ist.

10. Verfahren zum Herstellen eines probiotischen Produkts gemäß Anspruch 9, worin das probiotische Produkt ein Rohmaterial einer Molkerei ist, das unter Verwendung des KW3110-Stamms des Milchsäurebakteriums Lactobacillus paracasei oder seiner Mutante fermentiert ist.

11. Verfahren zum Herstellen eines probiotischen Produkts gemäß den Ansprüchen 9 bis 10, worin das probiotische Produkt ein Joghurt ist.

12. Verfahren zum Herstellen eines probiotischen Produkts gemäß den Ansprüchen 6 bis 11, worin die Anzahl des KW3110-Stamms des Milchsäurebakteriums Lactobacillus paracasei oder seiner Mutante im probiotischen Produkt 5 x 10⁹ oder mehr Zellen pro Tagesaufnahme ist.

13. Verfahren zum Herstellen eines probiotischen Produkts gemäß den Ansprüchen 6 bis 12, worin das probiotische Produkt zum Vorbeugen und/oder Behandeln von allergischen Erkrankungen verwendet wird.

14. Verwendung des KW3110-Stamms des Milchsäurebakteriums Lactobacillus paracasei oder seiner Mutante in der Herstellung eines probiotischen Produkts.

15. Verwendung des KW3110-Stamms des Milchsäurebakteriums Lactobacillus paracasei oder seiner Mutante in der Herstellung eines probiotischen Produkts gemäß Anspruch 14, worin das probiotische Produkt ein Getränk oder Nahrungsmittel ist.

16. Verwendung des KW3110-Stamms des Milchsäurebakteriums Lactobacillus paracasei oder seiner Mutante in der Herstellung eines probiotischen Produkts gemäß Anspruch 15, worin das probiotische Produkt ein Rohmaterial einer Molkerei ist, das unter Verwendung des KW3110-Stamms des Milchsäurebakteriums Lactobacillus paracasei oder seiner Mutante fermentiert ist.

17. Verwendung des KW3110-Stamms des Milchsäurebakteriums Lactobacillus paracasei oder seiner Mutante in der Herstellung eines probiotischen Produkts gemäß den Ansprüchen 15 bis 16, worin das probiotische Produkt ein Joghurt ist.

18. Verwendung des KW3110-Stamms des Milchsäurebakteriums Lactobacillus paracasei oder seiner Mutante in der Herstellung eines probiotischen Produkts gemäß den Ansprüchen 14 bis 17, worin die Anzahl des KW3110-Stamms des Milchsäurebakteriums Lactobacillus paracasei oder seiner Mutante im probiotischen Produkt 5 x 10⁹ oder mehr Zellen pro Tagesaufnahme ist.

19. Verwendung des KW3110-Stamms des Milchsäurebakteriums Lactobacillus paracasei oder seiner Mutante in der Herstellung eines probiotischen Produkts gemäß den Ansprüchen 14 bis 18, worin das probiotische Produkt zum Vorbeugen und/oder Behandeln von allergischen Erkrankungen verwendet wird.

## Revendications

1. Produit probiotique comprenant une bactérie d'acide lactique viable *Lactobacillus paracasei* souche KW3110 ou son mutant comme un ingrédient actif, dans lequel le produit probiotique est une boisson ou un produit alimentaire.

2. Produit probiotique selon la revendication 1, dans lequel le produit probiotique est une matière première laitière fermentée en utilisant la bactérie d'acide lactique *Lactobacillus paracasei* souche KW3110 ou son mutant.

3. Produit probiotique selon la revendication 1 à 2, dans lequel le produit probiotique est un yoghourt.

4. Produit probiotique selon la revendication 1 à 3, dans lequel le nombre de bactéries d'acide lactique *Lactobacillus paracasei* souche KW3110 ou de son mutant dans le produit probiotique est 5 x 10⁹ cellules ou plus par prise journalière.

5. Produit probiotique selon la revendication 1 à 4, dans lequel le produit probiotique est utilisé pour prévenir et/ou traiter des maladies allergiques.

6. Procédé de production de produits probiotiques, dans lequel une partie ou la totalité de la bactérie d'acide lactique dans la boisson ou l'aliment produit(e) en utilisant la bactérie d'acide lactique ou la boisson ou l'aliment comprenant la bactérie d'acide lactique est remplacée par la bactérie d'acide lactique *Lactobacillus* paracasei souche KW3110 ou son mutant.

7. Procédé de production d'un produit probiotique comprenant la bactérie d'acide lactique, dans lequel la bactérie d'acide lactique est *Lactobacillus paracasei* souche KW3110 ou son mutant.

8. Procédé de production d'un produit probiotique qui est produit en utilisant une bactérie d'acide lactique, dans lequel la bactérie d'acide lactique utilisée est *Lactobacillus paracasei* souche KW3110 ou son mutant.

9. Procédé de production d'un produit probiotique selon les revendications 6 à 8, dans lequel le produit probiotique est une boisson ou un produit alimentaire.

10. Procédé de production d'un produit probiotique selon la revendication 9, dans lequel le produit probiotique est une matière première laitière fermentée en utilisant la bactérie d'acide lactique *Lactobacillus paracasei* souche KW3110 ou son mutant.

11. Procédé de production d'un produit probiotique selon les revendications 9 à 10, dans lequel le produit probiotique est un yoghourt.

12. Procédé de production d'un produit probiotique selon les revendications 6 à 11, dans lequel le nombre de bactéries d'acide lactique *Lactobacillus paracasei* souche KW3110 ou de son mutant dans le produit probiotique est 5 x 10⁹ cellules ou plus par prise journalière.

13. Procédé de production d'un produit probiotique selon les revendications 6 à 12, dans lequel le produit probiotique est utilisé pour prévenir et/ou traiter des maladies allergiques.

14. Utilisation de la bactérie d'acide lactique *Lactobacillus paracasei* souche KW3110 ou de son mutant dans la fabrication d'un produit probiotique.

15. Utilisation de la bactérie d'acide lactique *Lactobacillus paracasei* souche KW3110 ou de son mutant dans la fabrication d'un produit probiotique selon la revendication 14, dans laquelle le produit probiotique est une boisson ou un produit alimentaire.

16. Utilisation de la bactérie d'acide lactique *Lactobacillus paracasei* souche KW3110 ou de son mutant dans la fabrication d'un produit probiotique selon la revendication 15, dans laquelle le produit probiotique est une matière première laitière fermentée en utilisant la bactérie d'acide lactique *Lactobacillus paracasei* souche KW3110 ou son mutant.

17. Utilisation de la bactérie d'acide lactique *Lactobacillus paracasei* souche KW3110 ou de son mutant dans la fabrication d'un produit probiotique selon les revendications 15 et 16, dans laquelle le produit probiotique est un yoghourt.

18. Utilisation de la bactérie d'acide lactique *Lactobacillus paracasei* souche KW3110 ou de son mutant dans la fabrication d'un produit probiotique selon les revendications 14 à 17, dans laquelle le nombre de bactéries d'acide lactique *Lactobacillus paracasei* souche KW3110 ou de son mutant dans le produit probiotique est 5 x 10⁹ cellules ou plus par prise journalière.

19. Utilisation de la bactérie d'acide lactique *Lactobacillus paracasei* souche KW3110 ou de son mutant dans la fabrication d'un produit probiotique selon les revendications 14 à 18, dans laquelle le produit probiotique est utilisé pour prévenir et/ou traiter des maladies allergiques.
